Europäisches Patentamt

European Patent Office

Office européen des brevets

(19)

(11) **EP 1 210 003 B1**

(12) **EUROPEAN PATENT SPECIFICATION**

(45) Date of publication and mention
of the grant of the patent:
**04.08.2004 Bulletin 2004/32**

(21) Application number: **01941464.8**

(22) Date of filing: **03.05.2001**

(51) Int Cl.[7]: **A61B 3/103**

(86) International application number:
**PCT/US2001/014448**

(87) International publication number:
**WO 2001/085016 (15.11.2001 Gazette 2001/46)**

(54) **OBJECTIVE MEASUREMENT AND CORRECTION OF OPTICAL SYSTEMS USING WAVEFRONT ANALYSIS**

OBJEKTIVE MESSUNG UND KORREKTUR VON OPTISCHEN SYSTEMEN MITTELS WELLENFRONTANALYSE

APPAREIL ET PROCEDE DE MESURE ET DE CORRECTION OBJECTIVES DE SYSTEMES OPTIQUES PAR ANALYSE DE FRONT D'ONDES

(84) Designated Contracting States:
**AT BE CH CY DE DK ES FI FR GB GR IE IT LI LU MC NL PT SE TR**

(30) Priority: **08.05.2000 US 566668**

(43) Date of publication of application:
**05.06.2002 Bulletin 2002/23**

(73) Proprietor: **Alcon, Inc**
**6331 Hünenberg (CH)**

(72) Inventors:
• **FREY, Rudolph, W.**
**Winter Park, FL 32789 (US)**
• **BURKHALTER, James, H.**
**Orlando, FL 32805 (US)**

• **ZEPKIN, Neil**
**Casselberry, FL 32718 (US)**
• **POPPELIERS, Edward**
**Orlando, FL 32804 (US)**
• **CAMPIN, John, A.**
**Orlando, FL 32828 (US)**

(74) Representative: **Hanna, Peter William Derek et al**
**Hanna, Moore & Curley,**
**11 Mespil Road,**
**Dublin 4 (IE)**

(56) References cited:
**WO-A-99/27334**

• **MACRAE S.: 'Guest Editorial' JOURNAL OF CATARACT AND REFRACTIVE SURGERY vol. 26, no. 2, February 2000,**

## Description

### Field of the Invention

The invention relates generally to optical aberration measurement and correction, and more particularly to an objective measurement and correction of optical systems, such as systems of a human eye.

### Background of the Invention

Optical systems having a real image focus can receive collimated light and focus it at a point. Such optical systems can be found in nature, e.g., human and animal eyes, or can be man-made, e.g., laboratory systems, guidance systems, and the like. In either case, aberrations in the optical system can affect the system's performance. By way of example, the human eye will be used to explain this problem.

A perfect or ideal eye diffusely reflects an impinging light beam from its retina through optics of the eye which includes a lens and a cornea. For such an ideal eye in a relaxed state, i.e., not accommodating to provide near-field focus, reflected light exits the eye as a sequence of plane waves. However, an eye typically has aberrations that cause deformation or distortion of reflected light waves exiting the eye. An aberrated eye diffusely reflects an impinging light beam from its retina through its lens and cornea as a sequence of distorted wavefronts.

There are a number of technologies that attempt to provide the patient with improved visual acuity. Examples of such technologies include remodeling of the cornea using refractive laser surgery or intra-corneal implants, adding synthetic lenses to the optical system using intra-ocular lens implants, and precision-ground spectacles. In each case, the amount of corrective treatment is typically determined by placing spherical and/or cylindrical lenses of known refractive power at the spectacle plane (approximately 1.0-1.5 centimeters anterior to cornea) and literally asking the patient which lens or lens combination provides the clearest vision. This is an imprecise measurement of true distortions in the reflected wavefront because 1) a single spherocylindrical compensation is applied across the entire wavefront, 2) vision is tested at discrete intervals (i.e., diopter units) of refractive correction, and 3) subjective determination by the patient is desired in order to determine the optical correction. Thus, conventional methodology for determining refractive errors in the eye is substantially less accurate than the techniques now available for correcting the ocular aberrations.

One method of measuring ocular refractive errors is disclosed in U.S. Patent No. 5,258,791 to Penney et al. for "Spatially Resolved Objective Autorefractometer," which teaches the use of an autorefractometer to measure the refraction of the eye at numerous discrete locations across the corneal surface. The autorefractometer is designed to deliver a narrow beam of optical radiation to the surface of the eye, and to determine where that beam strikes the retina using a retinal imaging system. Both the angle of the beam's propagation direction with respect to the optical axis of the system and the approximate location at which the beam strikes the corneal surface of the eye are independently adjustable. However, a small uncertainty or error in the location of the beam's point of incidence on the cornea exists due to the curved corneal surface. For each point of incidence across the corneal surface, the refraction of the eye corresponding to that surface point can be determined by adjusting the angle at which the beam strikes the cornea until the beam refracted on to the iris strikes the fovea centralis. Adjustment of the beam angle of propagation can be accomplished either manually by the patient or automatically by the autorefractometer, if a feedback loop involving a retinal imaging component is incorporated.

Penney '791 further teaches the use of the autorefractometer measurements in determining the appropriate corneal surface reshaping to provide emmetropia, a condition of a normal eye when parallel beams or rays of light are focused exactly on the retina and vision is perfect. This is accomplished by first obtaining an accurate measurement of corneal surface topography using a separate commercially available device. A mathematical analysis is then performed using an initial corneal topography at each surface reference point, the measured refraction at each surface point, and Snell's law of refraction, to determine a desired change in surface contour at each reference point. The contour changes at the various reference points are then combined to arrive at a single reshaping profile to be applied across the full corneal surface.

A major limitation to the approach described by Penney '791 is that a separate measurement of corneal topography is desired to perform the Snell's Law analysis of needed refraction change. This adds significantly to the time and cost of a complete and desirable diagnostic evaluation. Further, the accuracy of the refraction change analysis will be dependent upon the accuracy of the topographic measurement and the accuracy of the autorefractometer measurement. In addition, any error in the spatial orientation of a topography map with respect to a refraction map will degrade the accuracy of the needed correction profile. Yet another limitation to known approaches such as described in Penney '791, by way of example, is that test points on the corneal surface are examined sequentially. Eye motion during the examination, either voluntary or involuntary, could introduce substantial errors in the refraction measurement. Penney '791 teaches detection of such eye movement by deliberately including measurement points outside the pupil, i.e., in the corneal region overlying the iris, where the return from the retina will obviously be zero at specific intervals in the examination sequence. However, this approach may still allow substantial undetected eye movement error between such iris reference points.

By way of example, one method and system known in the art, are disclosed by Junzhong Liang et al. in "Objective Measurement Of Wave Aberrations Of The Human Eye With The Use Of A Hartmann-Shack Wave-Front Sensor," published in the Journal of the Optical Society of America, Volume 11, No. 7, July 1994, pages 1949-1957. Liang et al. teach the use of a Hartmann-Shack wavefront sensor to measure ocular aberrations by measuring the wavefront emerging from the eye by the retinal reflection of a focused laser light spot on the retina's fovea. The actual wavefront is reconstructed using wavefront-estimation with Zernike polynomials.

The imprecise measurement technique of placing lenses of known refractive power anterior to the comea and asking a patient which lens or lens combination provides the clearest vision has been improved with the use of autorefractometers, as described in Penny '79, or with the use of wavefront sensors as described by Liang et al. Spatially resolved refraction data, in combination with measured existing surface contour of the anterior surface of the eye, enable a calculation of a detailed spatially resolved new contour which provides corrected vision. However, it would be an improvement in this art if such vision correction could be made without the need for this contour data, and further without the need for feedback from the patient regarding an appropriate lens. Liang et al. discloses the use of a Hartmann-Shack wavefront sensor to measure ocular aberrations by measuring the wavefront emerging from the eye by retinal reflection of a focused laser light spot on the retina's fovea. A parallel beam of laser light passes through beam splitters and a lens pair which brings the beam to a focus point on the retina by the optics of the eye. Possible myopia or hyperopia of the tested eye is corrected by movement of a lens within the lens pair. The focused light on the fovea is then assumed to be diffusely reflected and acts as a point source located on the retina. The reflected light passes through the eye and forms a distorted wavefront in front of the eye that results from the ocular aberrations. The aberrated wavefront is then directed to the wavefront sensor.

A point source of radiation on the retina would be ideal for such measurements. However, when the perfect eye receives a collimated beam of light, the best possible image on the retina is a diffraction limited spot. As illustrated by way of example, with Penny et al. and Liang et al., discussed above, and typical for those of skill in the art, parallel or collimated beams are used with the optics of the eye being measured to achieve this diffraction limited spot for such objective measurements. To do so, a setup for each patient includes a corrective lens or lens combination and adjustments thereto for accommodating that patient's specific visual acuity. Providing a corrective or lens combination, as well as setting up for their use becomes cumbersome, time consuming, and at an additional expense. Eliminating the need for such corrective optics is desirable and eliminates a variable within optical measurement systems that typically include many variables. Further, there is a need for providing optical characteristics of an eye without requiring feedback from the patient. By way of example, the patient may be a wild or domestic animal, living or dead.

The Hartmann-Shack wavefront sensor disclosed by Liang et al. includes two identical layers of cylindrical lenses with the layers arranged so that lenses in each layer are perpendicular to one another, as further disclosed in U.S. Patent No. 5,062,702 to Bille. In this way, the two layers operate as a two-dimensional array of spherical lenslets that divide the incoming light wave into sub-apertures. The light through each sub-aperture is brought to focus in the focal plane of the lens array where a charge coupled device (CCD) image module resides.

The system of Liang et al. is calibrated by impinging an ideal plane wave of light on the lenslet array so that a reference or calibrating pattern of focus spots is imaged on the CCD. Since the ideal wavefront is planar, each spot related to the ideal wavefront is located on the optical axis of the corresponding lenslet. When a distorted wavefront passes through the lenslet array, the image spots on the CCD are shifted with respect to a reference pattern generated by the ideal wavefront. Each shift is proportional a local slope, i.e., partial derivatives of the distorted wavefront, which partial derivatives are used to reconstruct the distorted wavefront, by means of modal wavefront estimation using Zernike polynomials.

However, the system disclosed by Liang et al. is effective only for eyes having fairly good vision. Eyes that exhibit considerable myopia (nearsightedness) would cause the focus spots to overlap on the CCD, thereby making local slope determination practically impossible for eyes having this condition. Similarly, eyes that exhibit considerable hyperopia (farsightedness) deflect the focus spots such that they do not impinge on the CCD thereby again making local slope determination practically impossible for eyes having this condition.

## Summary of the Invention

The present invention relates to an optical correction system as defined in claim 1.

Preferred embodiments are detailed in the dependent claims.

## Brief Description of the Drawings

FIG. 1A is a schematic view of the ideal eye reflecting light from its retina as a planar wavefront;

FIG. 1B is a schematic view of an aberrated eye reflecting light from its retina as a deformed wavefront;

FIG. 1C is a schematic view of the distorted wavefront relative to a reference plane to show the wavefront error or optical path difference as a function of transverse distance in the propagation direction;

FIG. 1D is a schematic view illustrating use of a reference plane;

FIG. 2 is a simplified schematic of the system for determining ocular aberrations in accordance with the essential features of the present invention;

FIG. 3 is a schematic of a Hartmann-Shack wavefront analyzer;

FIG. 4 is a perspective view of a portion of the pinhole imaging plate and planar array of light-sensitive cells comprising the wavefront sensor from FIG. 3 where the deflection of a wavefront piece associated with an aberrated eye is shown in comparison with a wavefront piece associated with a calibration or planar wavefront;

FIG. 5 is a plan view of a designated area on the planar array of light-sensitive cells associated with a corresponding hole;

FIG. 6 is a schematic of another wavefront analyzer;

FIG. 7 is a schematic view of a prior art arrangement suitable for ophthalmic use;

FIG. 8 is a side view of a cornea showing a thickness of corneal material to be ablated as an optical correction;

FIG. 9 is a side elevation view of one embodiment of the present invention illustrating a patient positioning for measurement;

FIG. 10 is an end elevation view of the embodiment of FIG. 9;

FIG. 11 is an enlarged perspective view of an patient positioning portion of the embodiment of FIG. 9;

FIG. 12 is a top plan view of optical elements of the embodiment of FIG. 9;

FIG. 12A illustrates a fixation target optical path of FIG. 12;

FIG. 12B illustrates a video image optical path of FIG. 12;

FIG. 12C illustrates a probe laser optical path of FIG. 12;

FIG. 12D illustrates a re-emitted wavefront optical path of FIG. 12;

FIG. 12E illustrates a calibration wavefront optical path of FIG. 12;

FIGS. 12F and 12G are front elevation and top plan views of a trial lens holder useful with embodiments of the present invention herein described;

FIG. 13 is a block diagram illustrating electrical components of the embodiment of FIG. 9;

FIG. 14 is an enlarged image of an eye illustrating a centration image;

FIG. 15 is a block diagram illustrating an operable flow of steps used in one embodiment of the present invention;

FIG. 16 is an enlarged image of an eye illustrating a pre-measurement eye alignment;

FIG. 17 is an enlarged image of an eye illustrating a pre-measurement eye alignment checking thereof;

FIG. 18 is a line diagram illustrating an eye registration pattern;

FIG. 19 illustrates a rejected CCD image;

FIG. 20 illustrates a CCD image including centroids;

FIG. 21 is an enlarged image of a centroid;

FIG. 22 illustrates an image available to an operator of a measured and reference centroid;

FIG. 23A illustrates a spacial filter operable in one embodiment of the present invention;

FIG. 23B illustrates a noisy CCD image before filtering to provide an image as illustrated with reference to FIG. 20;

FIG. 24A is a three dimensional plot of a wavefront reconstruction in accordance with the present invention;

FIG. 24B illustrates a higher order aberration for the wavefront of FIG. 23;

FIG. 25 illustrates a geometric effect of a curved corneal surface on a wavefront measurement in accordance with the present invention;

FIGS. 26A and 26B illustrate ablation depth profiles for surgery on a myopic eye and a hyperopic eye, respectively, in accordance with the present invention;

FIG. 26C illustrates an ablation efficiency function for one embodiment of the present invention;

FIG. 27A is a pictorial line drawing illustrating magnification modification to the embodiment of FIG. 12; and

FIG. 27B is a pictorial line drawing illustrating optical elements of the present invention.

## Detailed Description of the Invention

The present invention will now be described more fully hereinafter with reference to the accompanying drawings, in which prior art examples and embodiments of the present invention are shown by way of illustration and example. This invention may, however, be embodied in many forms and should not be construed as limited to the embodiments set forth herein. Rather, these embodiments are provided so that this disclosure will be thorough and complete, and will fully convey the scope of the invention to those skilled in the art. Like numbers refer to like elements throughout.

By way of illustrative example, the present invention will be described with respect to diagnosing and correcting a human eye. However, it is to be understood that the teachings of the present invention are applicable to any optical system having a real image focus that can be, or can be adapted to diffusely reflect a focused spot of radiation from a rear portion of the optical system back through the optical system as a wavefront of radiation. Thus, the present invention can be used with human or animal eyes of patients that may be alive or dead, or any man-made optical system.

Correction of the human eye that may be used in conjunction with or based upon the diagnostic information provided by embodiments of the present invention include, by way of example, the grinding or preparation of eye glasses and lenses, which teachings are well known in the art, such as described in "Geometric, Physical, and Visual Optics" by Michael P. Keating, Ph.D. published by Butterworth Publishers, 80 Montvale Avenue, Stone, MA 02180, Copyright 1988. Laser surgery using lasers that photo ablate corneal tissue through the use of broad beam excimer lasers which are well known in the art, such as those disclosed in U.S. Patent No. 5,163,934 to Trokel, correction of presbyopia by photorefractive keratectomy disclosed in U.S. Patent No. 5,395,356 to King et al., and narrow beam systems as described in U.S. Patent No. 5,849,006 to Frey et al. in conjunction with a Lasik procedure which are well known in the art.

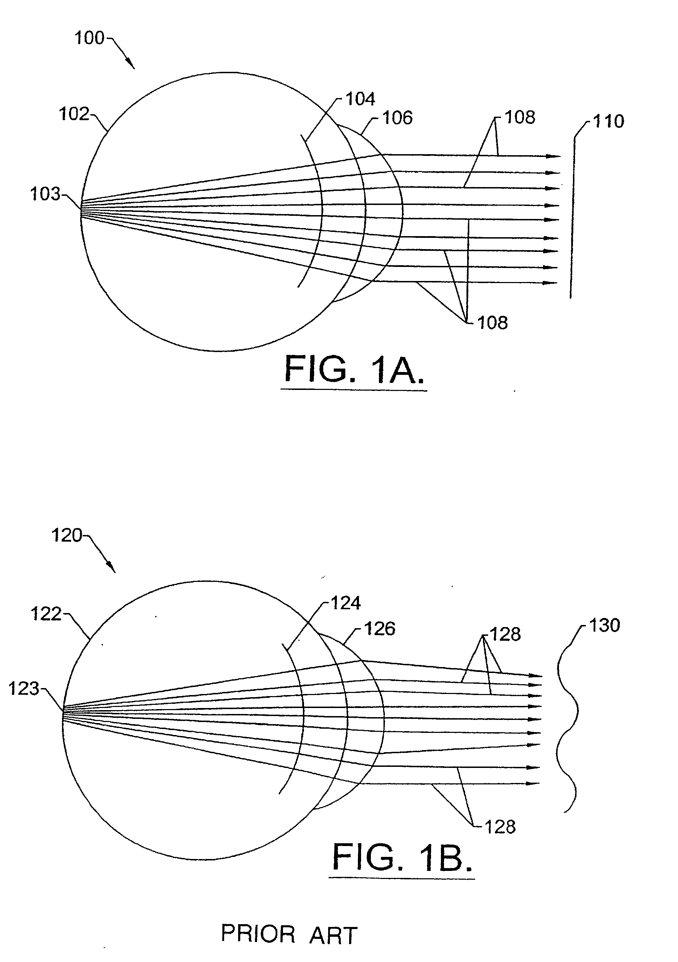

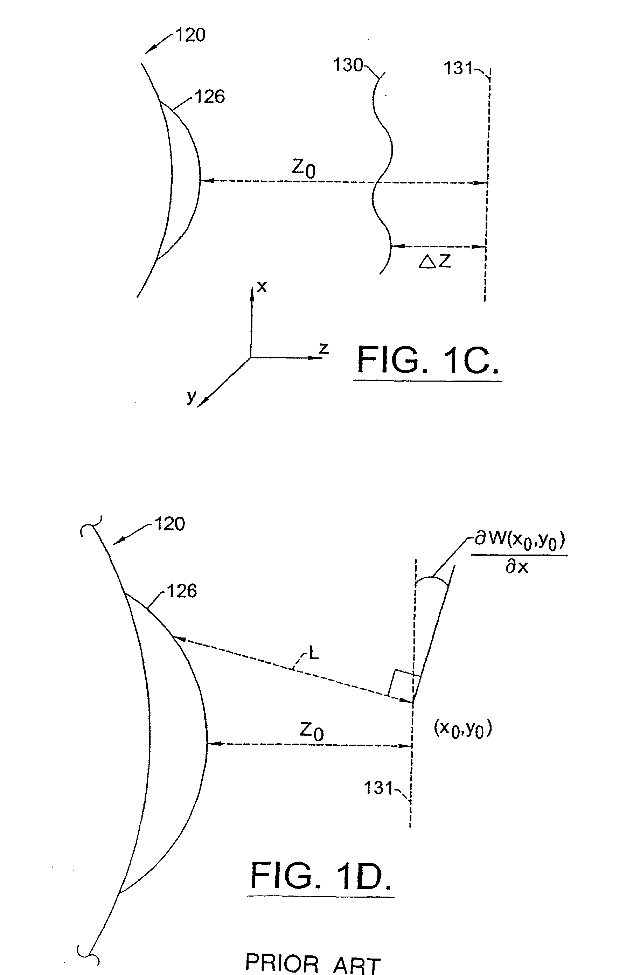

The method of using wavefront analysis to determine an appropriate optical correction will be introduced with reference to the eye example and the aid of the schematic drawings of FIGS. 1A, 1B, and 1C as disclosed in our International Patent Application No. WO-99/27334. As earlier described with reference to an ideal eye, and with reference now to FIG. 1A, the ideal emmetropic or perfect eye **100** diffusely reflects an impinging light beam (not shown for sake of clarity) from the back of its retina **102** (i.e., the fovea centralis **103**) through the eye's optics which includes lens **104** and cornea **106**. For such an ideal eye **100** in a relaxed state, i.e., not accommodating to provide near-field focus, the reflected light (represented by arrows **108**) exits the eye **100** as a sequence of plane waves, one of which is represented by straight line **110**. However, as illustrated with reference to FIG. 1B, a typical eye **120** normally has aberrations that cause deformation or distortion of a reflected wave exiting the eye, where the aberrated eye **120** diffusely reflects an impinging light beam (again not shown for sake of clarity) from the back of its retina **122** of the fovea centralis **123** through lens **124** and cornea **126**. For the aberrated eye **120**, the reflected light **128** exits the eye **120** as a sequence of distorted wavefronts, one of which is represented by wavy line **130**.

With reference now to FIG. 1C, a coordinate system is defined for convenience, where positive x is upward in the plane of the figure, positive y is outward from the plane of the figure, and positive z is to the right along a propagation direction. The distorted wavefront **130** is herein described mathematically as W(x,y). One method of measuring distortions in the wavefront **130** is by determining a spatial separation $\Delta z$ between a reference plane **131** (by way of example, a plane analogous to the ideal wavefront **110**) at a known distance $Z_0$ from the eye **120** at each (x,y) point of the distorted wavefront **130** as the leading edge of the wavefront **130** traverses the distance $Z_0$. This is described mathematically as:

$$\Delta Z(x,y) = Z_0 - W(w,y) \tag{1}$$

These $\Delta z$ measurements define optical path differences due to aberrations in the eye **120** being tested, by way of example. An appropriate correction consists of removing these optical path differences. By way of example, such correction is performed at reference plane **131**.

Depending on the desired corrective therapy (corneal tissue ablation, synthetic lens addition, by way of example), the amount of material removed or added at each (x, y) coordinate can be calculated directly if the refractive index of the material in question is known. For many procedures, such as intra-ocular lens implantation or radial keratotomy, a wavefront analysis may be performed repetitively during a procedure to provide feedback information as to the appropriate endpoint of the procedure.

In terms of the illustrative example, the differences $\Delta z(x,y)$ between the distorted wavefront **130** and the ideal wavefront **110** are the consequence of the aberrations in the eye. Correction of these aberrations consists of introducing an optical path difference at the reference plane **131** of negative $\Delta z(x,y)$. If the treatment approach, by way of example, consists of removing tissue from the surface of the cornea **126** by laser ablation, then one choice for the location of reference plane **131** is tangential to the surface of cornea **126** (i.e. at z=O). This is illustrated schematically with reference to FIG. 1D as disclosed in our International Patent Application No. WO-99/27334, where the curvature of the cornea **126** is greatly exaggerated for clarity of illustration. Ablation is then carried out discretely at each (x,y) coordinate along the cornea **126** by a laser beam delivery and eye tracking system such as described in U.S. Patent Nos.

5,980,513; 5,849,006; and 5,632,742, commonly owned with the present invention.

The appropriate corneal ablation depth at any (x,y) transverse coordinate is, to within a small error, given by:

$$\Delta z(x,y) / (n_c - 1) \qquad (2)$$

where $n_c$ is the refractive index of corneal tissue or 1.3775. The method described in detail below calculates $\Delta z(x,y)$ by first measuring the local slopes in wavefront **130**, i.e. $\partial W(x,y)/\partial x$ and $\partial W(x,y)/\partial y$, at a number of points in the transverse x and y directions in reference plane **131** and then generating a mathematical description of W(x,y) having slopes in best possible agreement with the experimentally determined values. One such slope $\partial W(x, y)/\partial x$ is illustrated with reference again to FIG. 1D. In doing this, a small error is introduced due to the fact that distorted wavefront **130** is measured at the reference plane **131** while wavefront **130** emerged from a curved corneal surface just posterior to reference plane **131**. By way of example, an error $E_x(x,y)$ is the lateral displacement in the x-direction at each (x,y) location at the measurement plane (i.e., reference plane **131**) to the curved corneal surface. A similar error will be manifest for any corrections involving curved optical surfaces. The error will generally increase with both (x,y) displacement from the point of tangency and local wavefront error.

For refractive surgery, the error may be negligibly small. The magnitude of error $E_x(x,y)$ can be found for each measurement location (x,y) measured at an arbitrary coordinate, e.g., $(x_0,y_0)$ by projecting that location back to the point of origin on the cornea **126**. This is explained mathematically with reference again to FIG. 1D, where by way of example, it is assumed that the error is in the plane of the figure, i.e., the plane defined by $y=y_0$, although it is quite straightforward mathematically to extend the analysis to include errors in the y-dimension. The quantification of a line L tracing the propagation of the wavefront **130** measured at $(x_0,y_0)$ in the $z_0$ reference plane from the corneal surface to the reference plane is:

$$L(x) = z_0 - \frac{(x - x_0)}{\partial W(x_0, y_0)/\partial x} \qquad (3)$$

If the corneal surface in the plane of the figure is described by the expression $S(x_0,y_0)$, then the point of origin for the wavefront **130** in question can be found by finding the point of intersection between L(x) and S (x, $y_0$). Mathematically, one finds the value x', that satisfies $L(x') = S(x_0,y_0)$. The error $E_x(x_0,y_0)$ is then given as $EX(X_0,Y_0)=X'-X_0$. Extending the analysis to consider errors in the y-direction would yield a similar expression for $E_y$ where $E_y(X_0, y_0)=y'-y_0$. If significant, these transverse errors can be compensated for by laterally displacing the aberration correction calculated at each (x,y) coordinate by the amounts $E_x(x,y)$ and $E_y(x,y)$.

In the case of human corneas, the transverse error under most circumstances will be negligible. The error will be zero at the origin where the corneal tissue and reference plane **131** are tangent. For human corneas, the tissue is approximately spherical with a radius of curvature of approximately 7.5-8.0 mm. The corrective treatment radius is typically no more than 3 mm, and local wavefront radius of curvature will almost never exceed 50 mm (a 20 diopter refractive error). The transverse error E at a 3 mm treatment radius for a local wavefront radius of curvature of 50 mm is less than 40 μm.

In order to perform wavefront analysis in a manner compatible with corrective procedures such as those described above, the amount of spatial separation of component portions of wavefront **130** relative to the corresponding component portions of the planar or ideal wavefront **110** is measured. It is the system and method of the present invention that allows such separation to be objectively and accurately measured for even substantially aberrated eyes **120** including those exhibiting severe defects such as severe myopia or hyperopia.

For the evaluation or measurement portion of the present invention, the patient's pupil should ideally be dilated to approximately 6 mm or more, i.e., the typical size of a human pupil in low light. Smaller amounts of dilation or no dilation at all may also evaluated or measured. In this way, the eye is evaluated while it is using the greatest area of the cornea so that any correction developed from such measurement takes into account the largest usable corneal area of the patient's eye. A lesser amount of the cornea is used in daylight where the pupil is considerable smaller, e. g., on the order of 3 millimeters. Dilation can be brought about naturally by implementing the measurement portion of the present invention in a low light environment such as a dimly lit room. Dilation can also be induced through the use of pharmacologic agents.

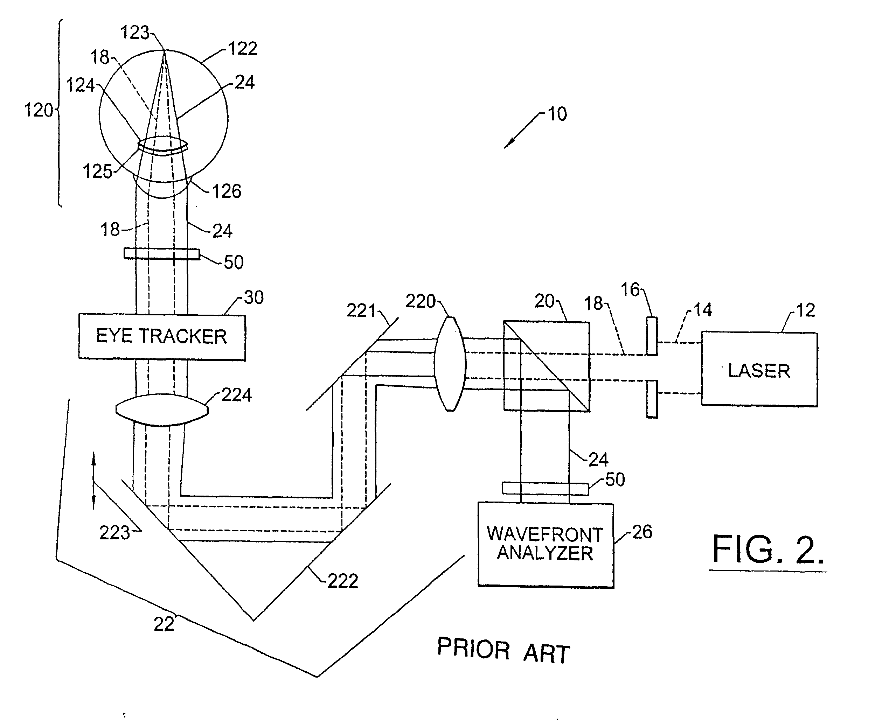

Referring now to FIG. 2 as disclosed in our International Patent Application No. WO-99/27334 a simplified schematic of one exemplary embodiment of the apparatus **10** is illustrated. The apparatus **10** includes a laser **12** for generating optical radiation used to produce a small-diameter laser beam **14**. The laser **12** generates a collimated laser light beam (represented by dashed lines for the beam **14**) of a wavelength and power that is eye-safe. For ophthalmic applications, appropriate wavelengths would include the entire visible spectrum and the near infrared spectrum.

To select a small-diameter collimated core of laser light beam **14**, an iris diaphragm **16** is used to block all of laser light beam **14** except for the laser beam **18** of a size desired for use. The laser beam **18** will have a diameter in the range of approximately 0.5-4.5 millimeters with 1-3 millimeters being typical, by way of example. A badly aberrated eye uses a smaller diameter beam while an eye with only slight aberrations can be evaluated with a larger diameter beam. Depending on the output divergence of the laser **12**, a lens, as will be later described, can be positioned in the beam path to optimize collimating of the beam.

Laser beam **18**, as herein described by way of example, is a polarized beam that is passed through a polarization sensitive beam splitter **20** for routing to a focusing optical train **22** which optical train operates to focus the laser beam **18** through the optics of the eye **120** (e.g., the cornea **126**, pupil **125** and the lens **124**) to the retina **122**. It is to be understood that the lens **124** may not be present for a patient that has undergone a cataract procedure. In the illustrated example of FIG. 2, the optical train **22** images the laser beam **18** as a small spot of light at or near the eye's fovea centralis **123** where the eye's vision is most acute. Note that the small spot of light could be reflected off another portion of retina **122** in order to determine aberrations related to another aspect of one's vision.

The diffuse reflection of the laser beam **18** back from the retina **122** is represented in FIG. 2 by solid lines **24** indicative of radiation that passes back through the eye **120**. The wavefront **24,** earlier described with reference to FIG. 1B as distorted wavefront **130** impinges on and is passed through the optical train **22** and on to the polarization sensitive beam splitter **20**. The wavefront **24** is depolarized relative to the laser beam **18** due to reflection and refraction as the wavefront **24** emanates from the retina **122**. Accordingly, the wavefront **24** is turned at the polarization sensitive beam splitter **20** and directed to a wavefront analyzer **26** such as a Hartmann-Shack (H-S) wavefront analyzer. In general, the wavefront analyzer **26** measures the slopes of wavefront **24**, i.e., the partial derivatives with respect to x and y, at a number of (x,y) transverse coordinates, as earlier described with reference to FIGS. 1C and 1D. This partial derivative information is then used to reconstruct or approximate the original wavefront with a mathematical expression such as a weighted series of Zemike polynomials.

Dynamic range enhancement is accomplished with the optical train **22** and/or a wavefront sensor portion of the wavefront analyzer **26**. With continued reference to FIG. 2, the optical train **22** includes a first lens **220,** a flat mirror **221**, a Porro mirror **222** and a second lens **224** all of which lie along the path of laser beam **18** and the wavefront **24**. The first lens **220** and the second lens **224** are identical lenses maintained in fixed positions. The Porro mirror **222** is capable of linear movement as indicated by arrow **223** to change the optical path length between the lenses **220** and **224**.

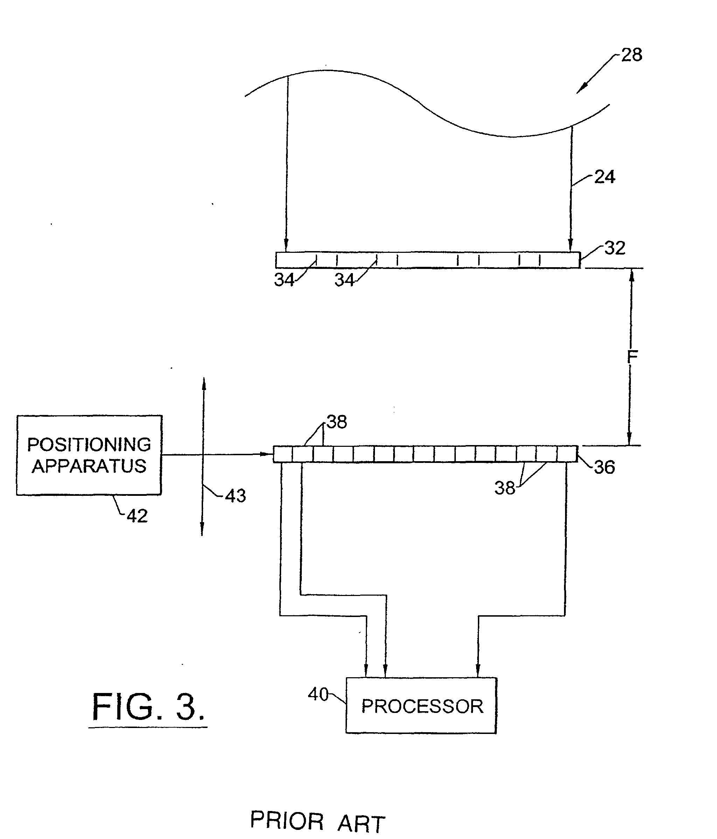

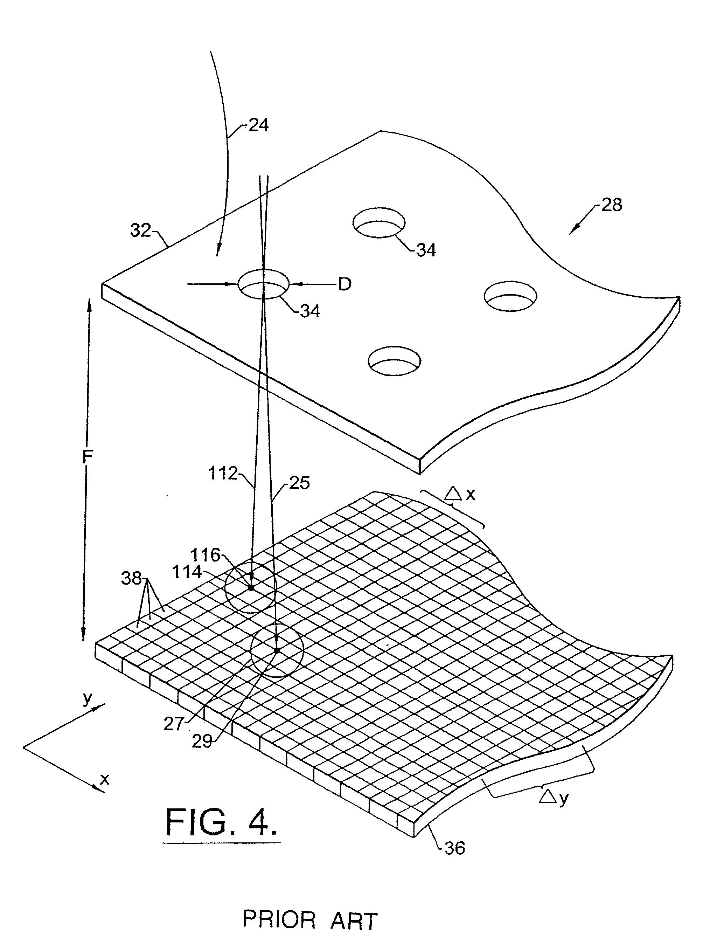

The dynamic range of the apparatus **10** is further improved by providing an improved wavefront sensor arrangement **28** as illustrated with reference to FIGS. 3 and 4 as disclosed in our International Patent Application No. WO-99/27334.

As illustrated with reference to FIG. 4 as disclosed in our International Patent Application No. WO-99/27334 when the wavefront **24** impinges on the plate **32**, a portion of the wavefront **24**, indicated by arrow **25**, passes through the hole **34** to illuminate planar array **36**. To a first order, the resulting image formed by each such wavefront portion **25** is a positive shadow of the respective hole **34**. However, diffraction occurs as determined by the diameter D of each hole **34,** the wavelength λ of the light source (e.g. the wavefront **24**) and the separation distance F between the plate **32** and the planar array **36.** The value of F is varied by the positioning apparatus **42** to adjust the gain based on the particular patient as will be explained further below.

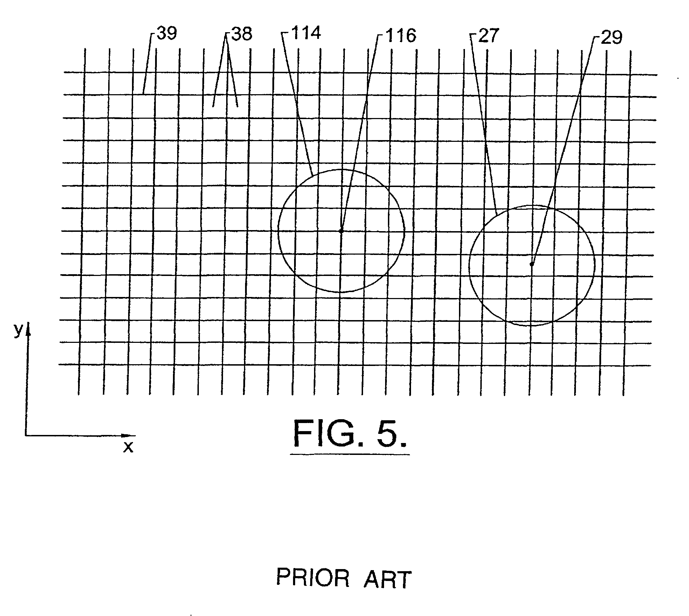

One method used to determine the centroid **29** of each spot **27** unambiguously with respect to a spot caused by another one of the holes **34**, assigns a unique number of cells **38** to each hole **34**. The "assigned areas" are designated, as illustrated with reference to FIG. 5, as also described in our International Patent Application No. 99/27334 by way of example, with the heavy grid lines **39**. It is to be understood that the grid lines **39** are not actual physical boundaries between cells **38** but are shown simply to illustrate the unique designated areas containing a plurality of the cells **38**. It is anticipated that other centroid strategies will be utilized that do not necessitate such partitioning of the array **36** given the teachings of the present invention.

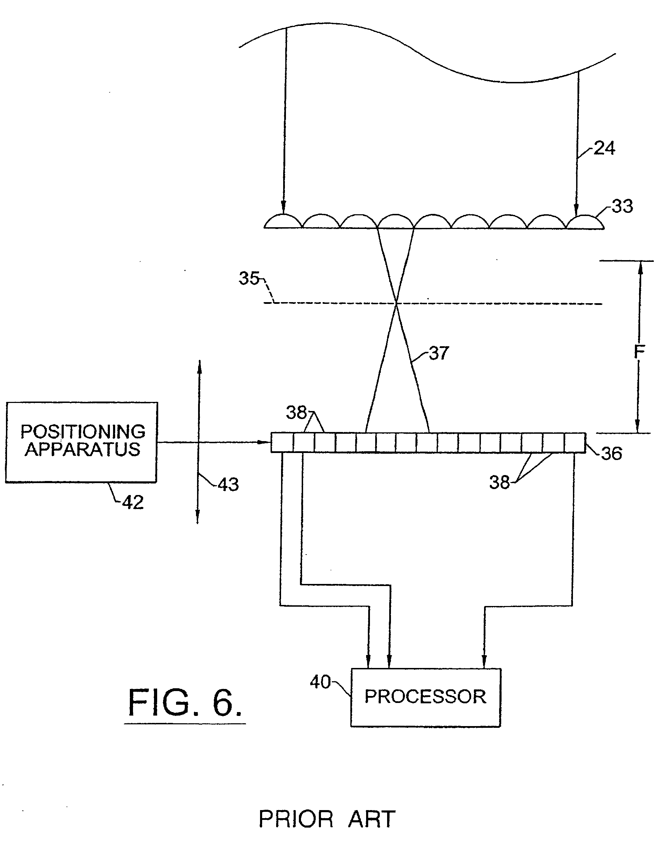

By way of example, a wavefront analyzer that replaces plate **32** described with reference to FIG. 3, with a two dimensional array of identical spherical lenslets **33**, could be used, as illustrated with reference to FIG. 6 as also described in our International Patent Application No. 99/27334 in such an arrangement, the lenslet array **33** may be operable by the positioning apparatus **42** such that separation distance F is independent of the focal length f that defines the focal plane of the lenslet array **33** which is represented by dashed line **35**.

Regardless of the structure of the wavefront sensor, the processor **40** computes each two-dimensional centroid **29** of each spot **27** generated by the wavefront **24**. The amount of two dimensional centroid shift relative to the centroid of the calibrating spot for each designated area associated with a corresponding hole **34** (or sub-aperture of lenslet array **33**) is divided by the separation distance F to generate a matrix of local slopes of the wavefront, i.e., $\partial W$

(x,y)/∂x and ∂W(x,y)/∂y at the (x,y) coordinates of the centers of holes **34**. For simplicity of discussion, these will be indicated by P(x,y)=∂W(x,y)/∂x and Q(x,y)=∂W(x,y)/∂y, respectively.

Numerous methods exist for using the partial derivative data to calculate the distorted wavefront **130** and **24** as described above with reference to FIGS. 1B and 2. By way of example, such approaches may include the use of Fourier series and Taylor series.

$$W(x, y) = \sum_{i=0}^{n} C_i Z_i(x, y) \qquad (4)$$

Briefly, the wavefront W(x,y) is expressed as a weighted sum of the individual polynomials where $C_i$ are the weighting coefficients, and $Z_i(x,y)$ are the Zernike polynomials up to some order. This is further described in our International Application WO 9927334.

A method is herein described in accordance with the present invention for identifying individual spots and correlating their geometry. The apparatus is configured such that the optical axis is aligned to the center of a particular aperture at the entrance face of the wavefront sensor. This aperture is located at or near the center of the entrance face. If the probe beam entering the eye is also aligned to the system optical axis, then due to the reversible nature of light rays, a light spot will always be seen directly behind the aligned aperture. That is, a spot will always be seen on the CCD sensor at this location, regardless of the wavefront aberrations, and will always correspond to the overlying aperture. Immediately adjacent spots will be minimally displaced from their "zero slope" locations. As one moves further from the central reference spot, generally greater spot displacements will occur. Using this knowledge, it is a relatively straight forward process to identify all the spots in the CCD pattern and establish their geometric relationships.

The displacement of the centroid from that of a perfectly collimated light beam, corresponding to ideal and emmetropic vision, is then calculated and used to determine the wavefront slope at each sample location. The location of the centroids for a collimated light beam may either be directly measured in a calibration step prior to the patient exam, or taken from a calculated reference pattern based on the wavefront sensor construction.

Multiple exposures may be used to check for improper eye alignment or eye movement during individual exposures. If eye movement during exposures cannot be analyzed successfully by acquiring multiple exposures, then the apparatus **10** can be augmented by the addition of an eye tracker **25**, illustrated with reference again to FIG. 2. One possible placement of the eye tracker **25** is herein illustrated. However, it is to be understood that the eye tracker **25** could be placed elsewhere within the apparatus **10**. One such eye tracker is disclosed in the aforementioned U.S. Patent No. 5,980,513, commonly owned with the present invention. In this way, wavefront analysis is performed even during a limited amount of eye motion.

A one-time calibration exposure can also be used to determine the relative sensitivities of the individual cells. This is made in uniform collimated light with plate **32** removed. The responses of individual cells are then recorded. For each light transmissive aperture (e.g, hole **34**), the centroid in the collimated case serves as a dedicated origin for the particular hole. The shift from the "origin" for each hole to the centroid caused by the wavefront **24** (as observed in this coordinate system) is determined by the direction of the wave surface corresponding to that hole. If Δx(m,n) is the x-component of the (m,n)th centroid and F is the plate separation, then the P-value for the (m,n)th centroid is:

$$P(m,n) = \partial x(m,n)/\partial z = \Delta x(m,n)/F \qquad (6)$$

The corresponding expression for Q is:

$$Q(m,n) = \partial y(m,n)/\partial z = \Delta y(m,n)/F \qquad (7)$$

Thus, each P(m,n) and Q(m,n) represents the partial derivatives of W(x,y) with respect to x and y for the (x,y) coordinates of each hole **34.** For an m-order Zernike approximation of the original wavefront, the experimentally determined P's and Q's are then used in the following equations to calculate the appropriate $C_i$ weighting coefficients as follows:

$$P(m,n) \;=\; \frac{\partial W(x,y)}{\partial x} \;=\; \sum_{i=0}^{n} C_i \frac{\partial Z_i(x,y)}{\partial x} \qquad\qquad \textbf{(8)}$$

$$Q(m,n) \;=\; \frac{\partial W(x,y)}{\partial x} \;=\; \sum_{i=0}^{n} C_i \frac{\partial Z_i(x,y)}{\partial x} \qquad\qquad \textbf{(9)}$$

By using a least-squares approx(m,n)/$\partial$zach to minimize the error between the actual wavefront slopes on the left hand side in the above equations and the Zemike approximations on the right hand side, optimal values for the weighting coefficients can be obtained.

In one possible approach to calculating a centroid $(x_c, y_c,)$, each hole **34** is assigned its dedicated area of the array **36** or $(I_{m,n} \pm \Delta i, j_{m,n} \pm \Delta j)$. This square of many light-sensitive cells is large enough that neighboring hole images never encroach, and all illumination from this hole is contained. The square contains $4\Delta i^*\Delta j$ cells.

If array 36 is designated $C_{k,1} = (x_c(i, j), y_c, (i, j))$, k, 1 = 0 ... 2$\Delta$1, 2$\Delta$j, and the spacing on centers is $\Delta x = \Delta y = d$, the measured cell responses are V (k,1) and the relative responsivities are R (k, l), then the x-component $x_c$, a function of i, j is represented by

$$x_c(i,j) = \left[ \sum_{k,1} V(k,1) * R(k,1) * d * k \right] \;/\; \left[ \sum_{k,1} V(k,1) * R(k,1) \right] \qquad \textbf{(10)}$$

and the y-component $y_c$, as a function of i,j is represented by

$$y_c(i,j) = \left[ \sum_{k,1} V(k,1) * R(k,1) * d * 1 \right] \;/\; \left[ \sum_{k,1} V(k,1) * R(k,1) \right] \qquad \textbf{(11)}$$

Then, if $(x_{c0}(i, j), y_{c0}(i, j))$ is the "origin centroid" for the (i, j) hole, i.e., made in perpendicular collimated light, and $(x_{cw}(i, j), y_{cw}(i, j))$ is the corresponding centroid found for the wavefront to be measured, then the relative centroid shift $(x_{cr}(i,,j)), Y_{cr}(i,j))$ is found as

$$x_{cr}(i,j) = x_{cw}(i,j) - x_{c0}(i,j) \qquad\qquad \textbf{(12)}$$

$$y_{cr}(i,j) = y_{cw}(i,j) - y_{c0}(i,j) \qquad\qquad \textbf{(13)}$$

The values P(i,j) and Q(i,j) are determined from

$$P(i,j) = x_{cr}(i,j) / F \qquad\qquad \textbf{(14)}$$

and

$$Q(i,j) = y_{cr}(i,j) / F \qquad (15)$$

The surface partial derivatives P(i,j) and Q(i,j) for the array of hole centers of plate 32 are next used to calculate the appropriate Zemike polynomial weighting coefficients to describe the original wavefront W(x,y). This will now be explained by way of illustration for a 7 x 7 square array of holes 34. However, it is to be understood that other sizes and shapes of hole arrays could be used.

First, a 1 x 98 matrix (i.e., column vector) PQ(k) is formed as

$$PQ(k) = P(7i + j),\ j = 0\ldots6,\ i = 0\ldots6,\ k = 0\ldots48 \qquad (16)$$

$$PQ(k) = Q(7i + j),\ j = 0\ldots6,\ i = 0\ldots6,\ k = 49\ldots98 \qquad (17)$$

with j cycling for each i, i.e., PQ (18) = P (2,5).

The matrix PQ is multiplied from the left with a transition matrix TM to get the matrix C as follows

$$C = TM*PQ \qquad (18)$$

where TM is a 98 wide by 14 high matrix and C is a 1 wide by 14 high matrix or column vector. C is the matrix $C_k$ k=1, ...,14 such that, to a least square error,

$$W(x,y) = \sum_k C_k * Z_k(x,y) \qquad (19)$$

and TM is calculated for a given aperture, e.g., a 6 millimeter pupil aperture. The functions $Z_k$ (x,y) in equation (19) are the Zernike polynomials. There is no standard convention as to their sequence. Thus, for consistency, it is important that the same sequence is used to produce the set $C_k$ that was chosen for deriving the matrix TM. They occur in groups of the same order, which is the highest exponent in the group, with the total number of members in an order increasing with the order. For example, in a fourth order analysis, orders up to and including 4 are used (less $Z_0$ - the single member of order 0 that is the constant 1 which describes the reference position of the group in the z direction). Since wavefront 24 is moving along z (at the velocity of light), this "piston term" describes only an arbitrary offset in Z, and this term may be ignored. The first 5 orders (0, 1, ...,4) contain 15 functions including the piston term.

Thus, in the illustrated example, 14 values of $C_k$ are calculated as coefficients of 14 Zernike polynomials. By way of example, one such order used to calculate TM is herein illustrated, and includes both the Zernike functions and their partial derivatives.

**ZERNIKE (X,Y) POLYNOMIAL EXPANSION THROUGH ORDER 4**

Polynomial Order 0

```
Z(0)        +1
dZ(0)/dx    0.0
DZ(0)/dy    0.0
```

Polynomial Order 1

Z(1)      +y
dZ(1)/dx   0.0
dZ(1)/dy   +1
Z(2)      +x
dZ(2)/dx   +1
dZ(2)/dy   0.0

Polynomial Order 2

Z(3)      $-1 +2y^2 +2x^2$
dZ(3)/dx   +4x
dZ(3)/dy   +4y
Z(4)      +2xy
dZ(4)/dx   +2y
dZ(4)/dy   +2x
Z(5)      $-y^2+x^2$
dZ(5)/dx   +2x
dZ(5)/dy   -2y

Polynomial Order 3

Z(6)      $-2y+3y^3+3x^2y$
dZ(6)/dx   +6xy
dZ(6)/dy   $-2+9y^2+3x^2$
Z(7)      $-2x+3xy^2+3x^3$
dZ(7)/dx   $-2+3y^2+9x^2$
dZ(7)/dy   +6xy
Z(8)      $-y^3+3x^2y$
dZ(8)/dx   +6xy
dZ(8)/dy   $-3y^2+3x^2$
Z(9)      $-3xy^2+x^3$
dZ(9)/dx   $-3y^2+3x^2$
dZ(9)/dy   -6xy

Polynomial Order 4

Z(10)      $+1-6y^2+6y^4-6x^2+12x^2y^2+6x^4$
dZ(10)/dx   $-12x+24xy^2+24x^3$
dZ(10)/dy   $-12y+24y^3+24x^2y$
Z(11)      $-6xy+8xy^3+8x^3y$
dZ(11)/dx   $-6y+8y^3+24x^2y$
dZ(11)/dy   $-6x+24xy^2+8x^3$
Z(12)      $+3y^2-4y^4-3x^2+4x^4$
dZ(12)/dx   $-6x+16x^3$
dZ(12)/dy   $+6y-16y^3$
Z(13)      $-4xy^3+4x^3y$
dZ(13)/dx   $-4y^3+12x^2y$
dZ(13)/dy   $-12xy^2+4x^3$

Z(14)       $+y^4-6x^2y^2+x^4$
dZ(14)/dx   $-12xy^2+4x^3$
dZ(14)/dy   $+4y^3-12x^2y$

The choice of sequencing the Zernike polynomials dictates the interpretations of the $C_k$ in equation (19) and therefore the order of terms in the TM matrix. Hence, the TM matrix is calculated after the choice is made. The development of the TM matrix for the illustrated example will be explained below.

Note that the fourth order analysis is only an example and is not the only possibility. A Zemike analysis can be done to any order. In general, the higher the order, the more accurate the result over the tested points. However, an exact polynomial fit over the tested points is not necessarily desirable. Such fits have the typical disturbing property that, unless the surface itself happens to be an exact polynomial of order no higher than that used for the surface fit, forcing an exact fit at separated points often causes wild swings between fitted points. That is, in polynomial surface fitting, an exact fit at a finite number of points can yield a poor average fit for a general function.

Calculation of the $\Delta z$ (x,y) optical path difference information from the Zernike reconstruction of the wavefront is accomplished simply by subtracting a constant from the Zemike approximation. The value of the constant will depend on the desired characteristics of $\Delta z$ (x,y). Depending on the method chosen to correct the aberrations (e.g., laser ablation, lens addition, etc.) it may, for example, be desirable to set either the maximum, mean or minimum value in $\Delta z$ (x,y) equal to zero.

The development of the transition matrix TM will now be explained for the illustrated example of a 7 x 7 array of holes in plate 32. At each point $(x_i, y_j)$, the tangents of the components of the normal are P $(x_i, y_j)$ and Q $(x_i, y_j)$ where

$$P\left(x_i, y_j\right) = \partial W\left(x_i, y_j\right) / \partial x \qquad (20)$$

and

$$Q\left(x_i, y_j\right) = \partial W\left(x_i, y_j\right) / \partial y \qquad (21)$$

Combining these with equation (11),

$$P\left(x_i, y_j\right) = \sum_k C_k \partial W\left(x_i, y_j\right) / \partial x \qquad (22)$$

and

$$Q\left(x_i, y_j\right) = \sum_k C_k \partial W\left(x_i, y_j\right) / \partial y \qquad (23)$$

each applicable to 49 (i,j) combinations. These are combined into a single column vector PQ that is 98 elements high, i.e., a 98 x 1 matrix. Defining two matrices $C_k$ (14 high x 1 wide) and $M_{k,(i,j)}$ (14 wide x 98 high)

$$\left(M_{k,(i,j)}\right) = \partial Z_k\left(x_i, y_j\right) / \partial x \quad ; \quad \partial Z_k\left(x_i, y_j\right) / \partial y \qquad (24)$$

where the x-derivatives are the first 49 rows and the y-derivatives are the last 49 rows. Then, equation (19) can be rewritten as the matrix equation

$$(PQ) = (M)(C) \qquad (25)$$

where the top 49 rows of M are the $\partial W(x_i, y_j)/\partial y$.

The expression in equation (25) gives the normal components in terms of the Zernike coefficients for a surface described by the array of 14 C's. These are exact, but it is not guaranteed that the actual total surface can be described by such an array of coefficients. Accordingly, if it is assumed that the description is within an acceptable tolerance, i. e., tolerating the errors that remain after least square error determination, then equation (26) can be considered to define the column vector C implicitly in terms of the mathematical matrix M and the measured vector PQ, both of which are known. The method of effecting the solution under the minimization condition is as follows. First, equation (25) is multiplied on the left by $M^T$, the transpose of M such that

$$(M^T)(PQ) = (M^T)(M)(C) = (S)(C) \qquad (26)$$

where

$$S \equiv M^T M \qquad (27)$$

is a square and symmetric matrix, e.g., of dimensions 14 x14 (with each element the sum of 98 products). Such a matrix has an inverse unless the determinant of its coefficients is zero. Since this is based on the Zernike polynomials alone, and they are all independent of each other, the determinant is non-zero, so that an inverse $S^{-1}$ is defined. Next, equation (25) is multiplied on the left by $S^{-1}$ to yield

$$(S^{-1})(M^T)(PQ) = (S^{-1})(S)(C) = (I)(C) = C \qquad (28)$$

Then, the mathematical transition matrix (independent of measurement) is

$$(TM) = (S^{-1})(M^T) \qquad (29)$$

and the "best fit" array of C's from the measured PQ's can be produced by the simple matrix multiplication

$$(C) = (TM)(PQ) \qquad (30)$$

To evaluate the eye unambiguously, all spots illuminating the planar array **36** due to a wavefront **24** are incident on the planar array simultaneously. If it is desired to reduce effects of eye movement, a pulsing or shuttering laser source may be used, or an eye tracker.

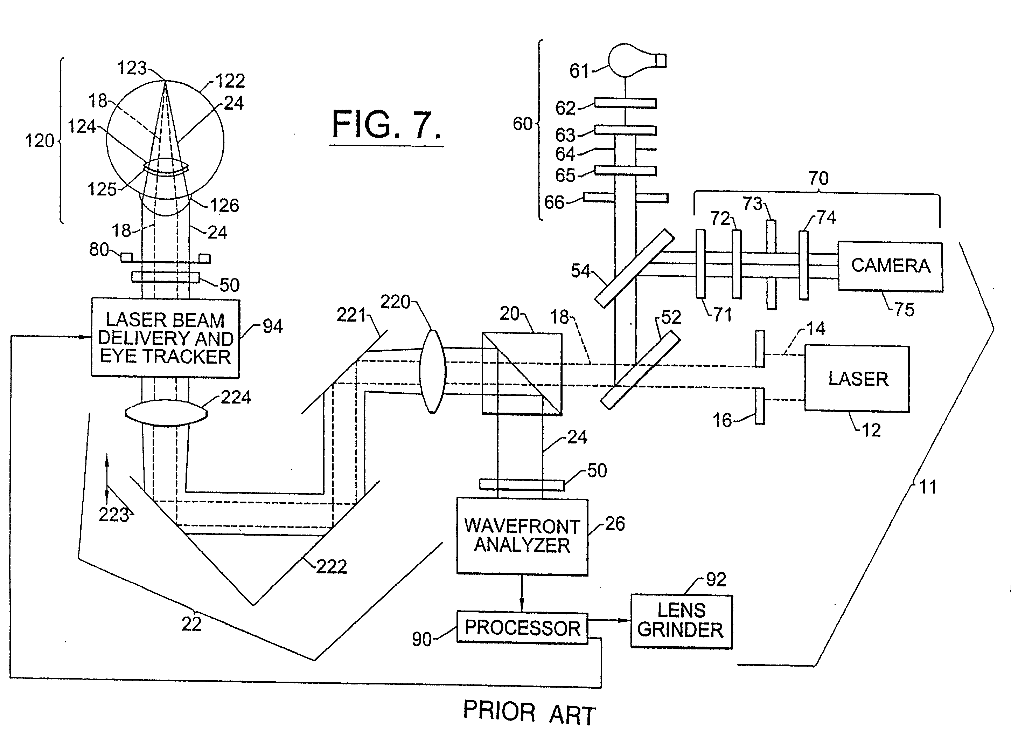

An implementation suitable for clinical use is illustrated, by way of example, with reference to FIG. 7 as also described in our International Patent Application No. 99/27334 and is referenced generally by numeral **11**. Like reference numerals are used to describe elements that are the same as those described above with respect to the apparatus **10**. A dichroic beam splitter **52** is interposed between the beam splitter **20** and the optical train **22** to introduce fixation target optics **60** and observation optics **70** into the apparatus **11** which are optically separated from one another by a 50/50 beam splitter **54**. Fixation target optics provide the eye **120** with visible light in the shape of a target. The visible light generated by fixation target optics **60** is reflected by the dichroic beam splitter **20** and directed through optical train **22**.

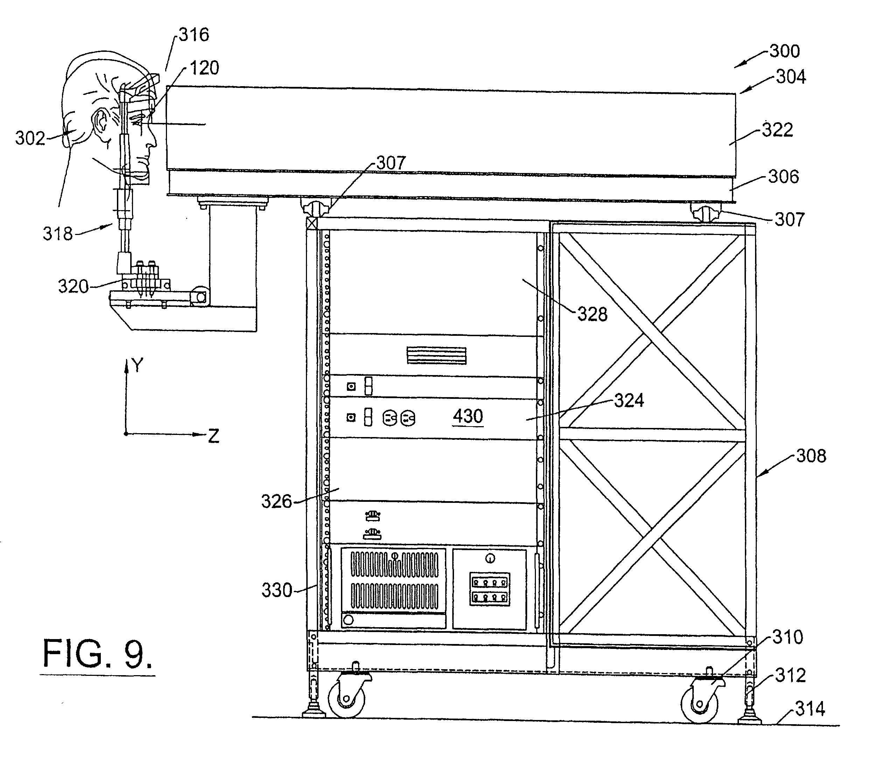

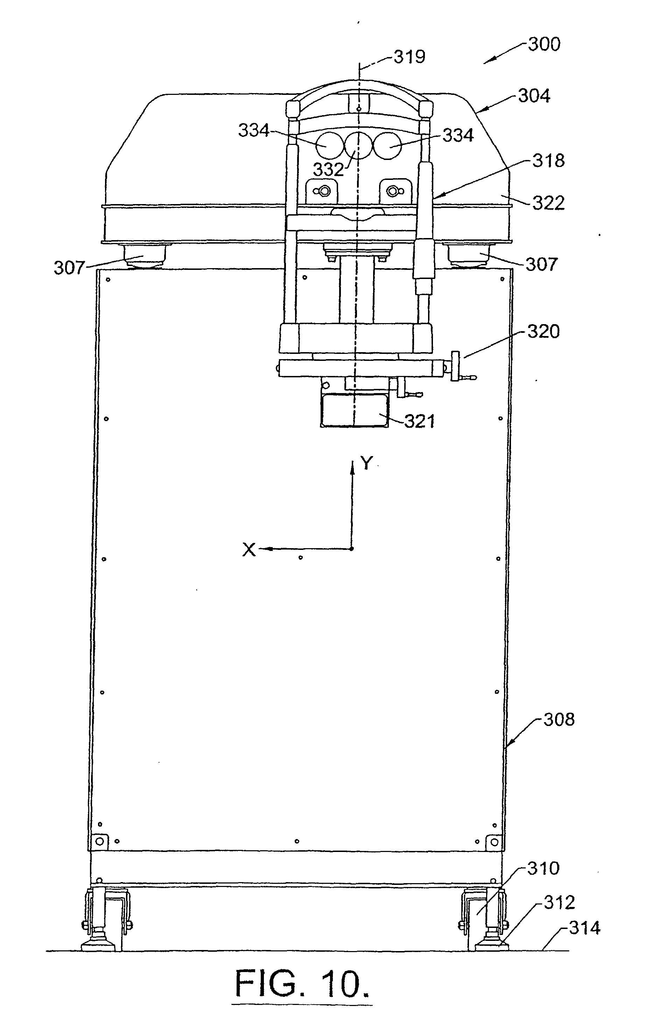

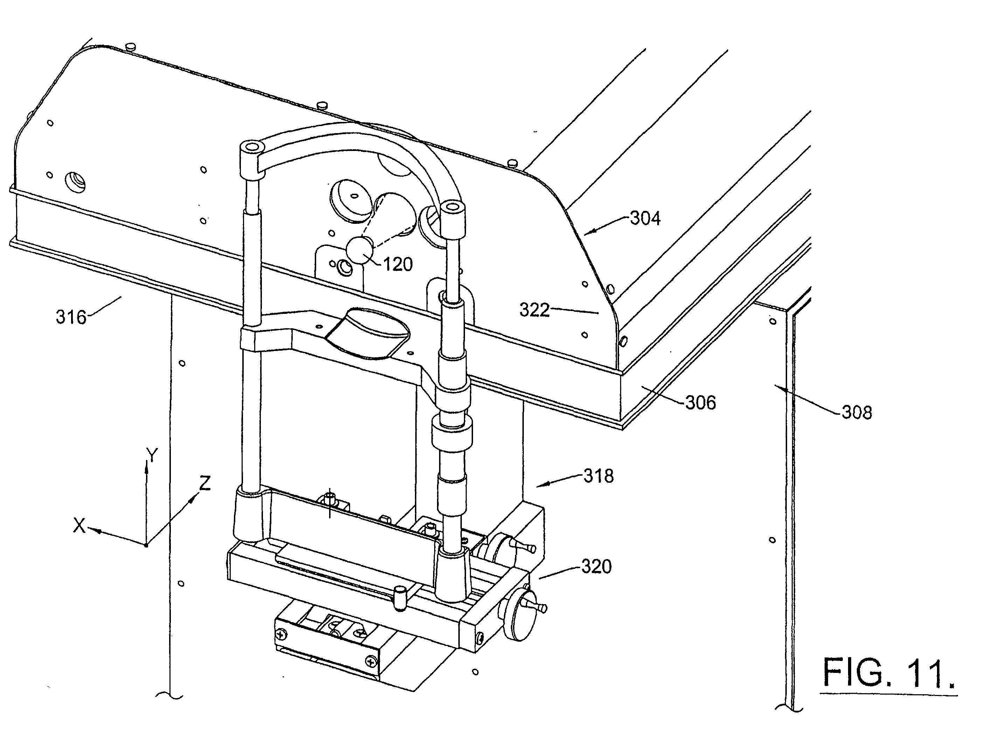

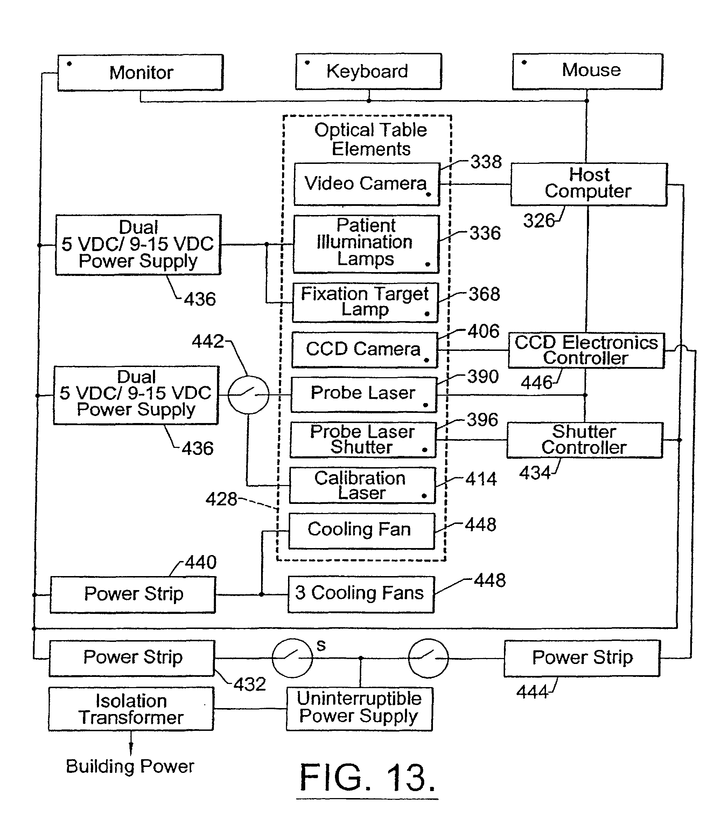

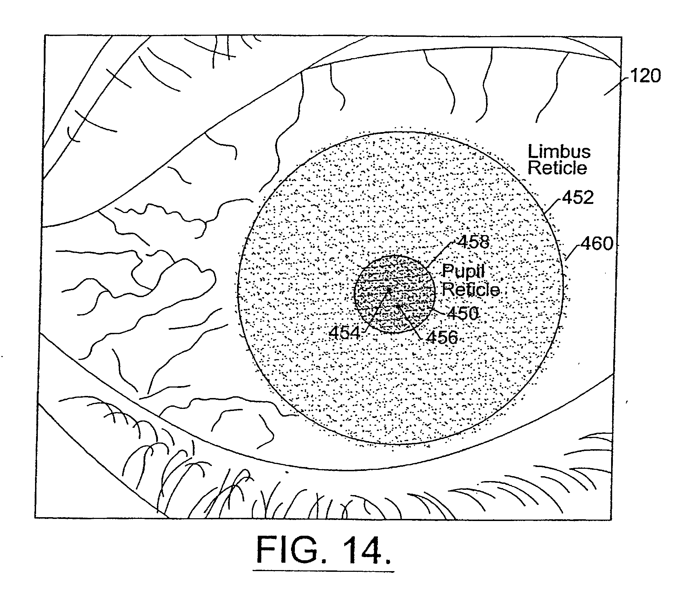

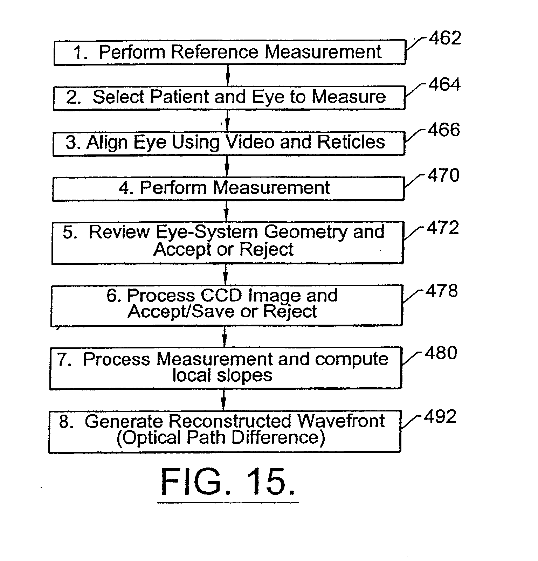

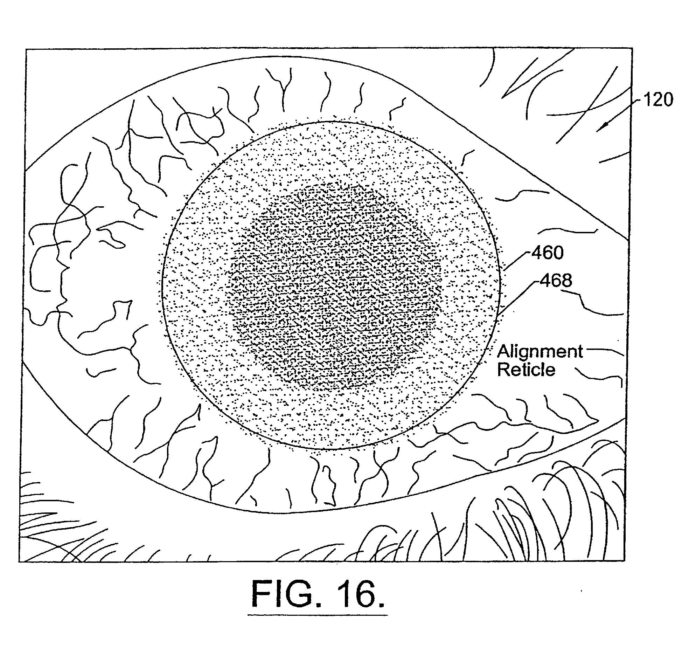

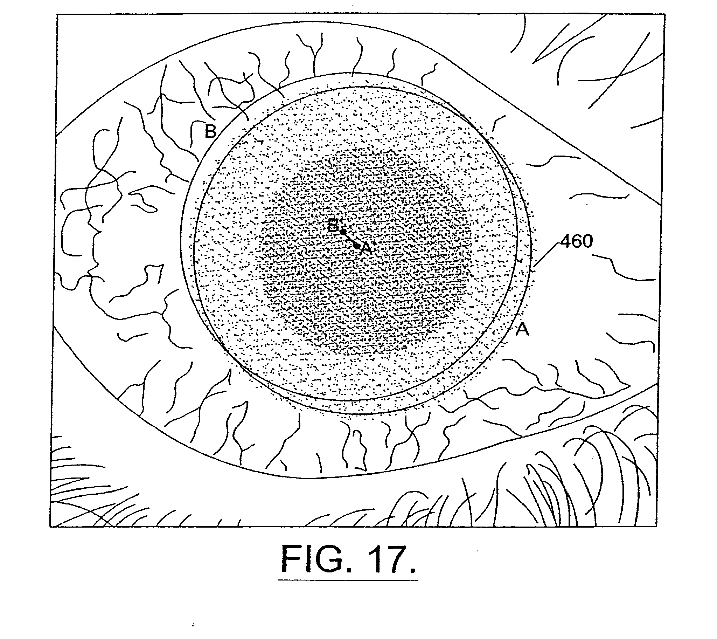

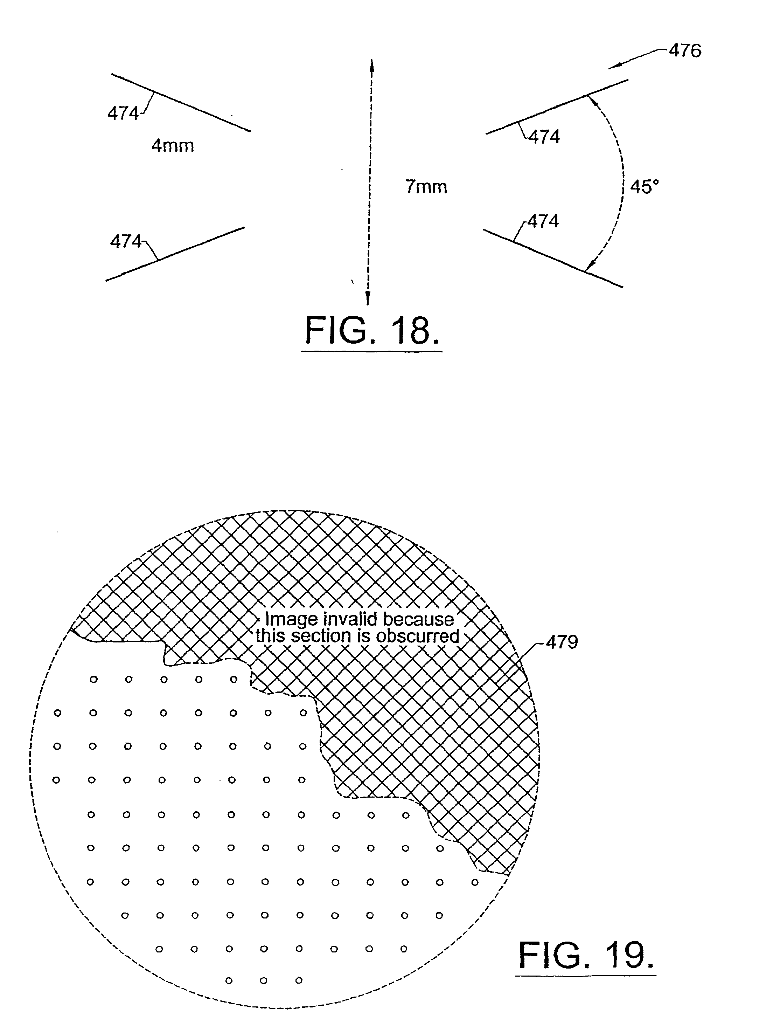

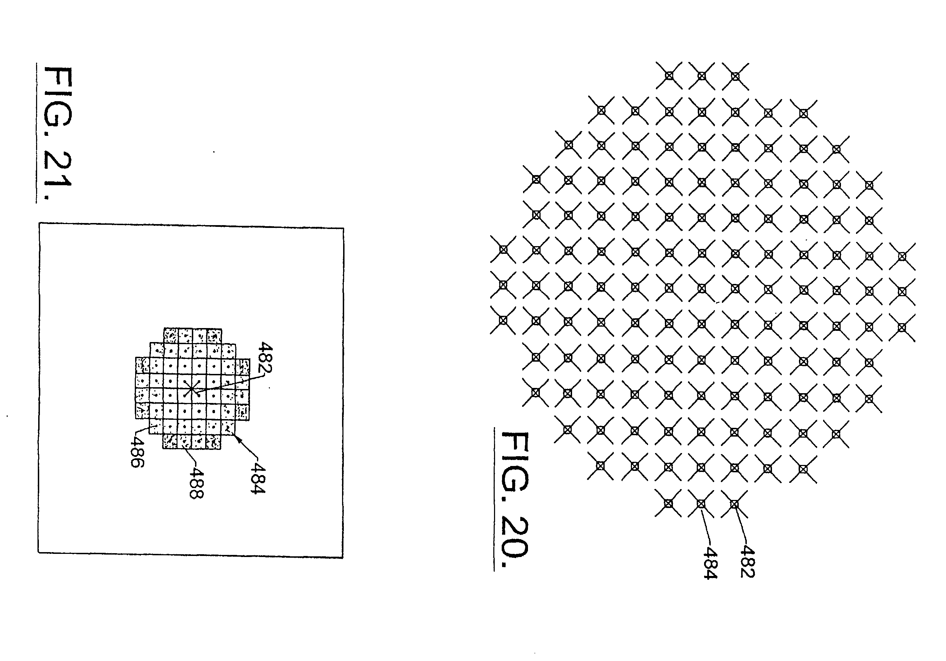

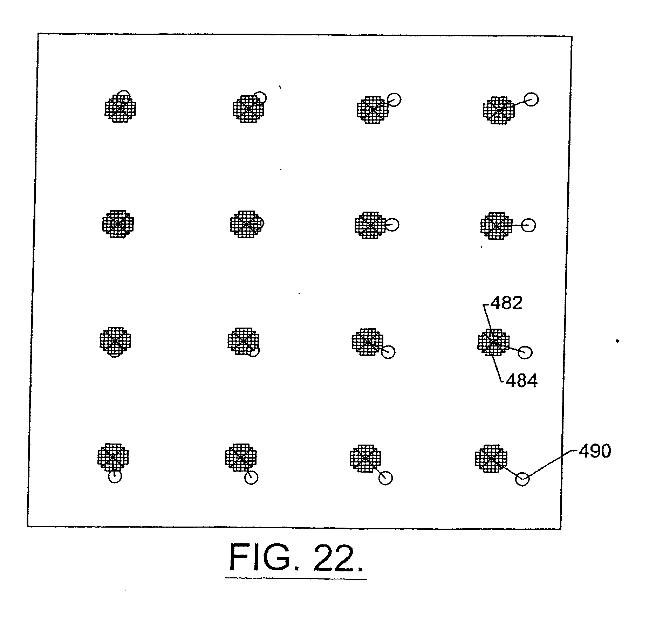

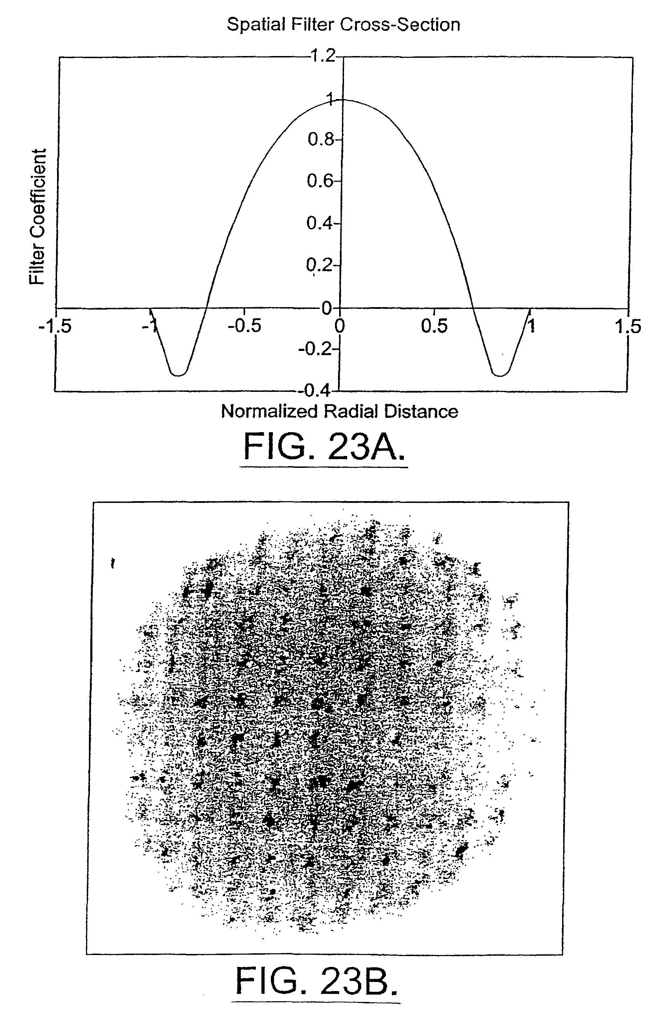

With reference now to FIGS. 9-24B and 17A, 27B also referred to in our co-pending European Patent Application No. EP-A-1,153,570, an exemplary embodiment of the apparatus **10** will be herein described beginning with series **300**, which improved apparatus **300** is constructed as a patient examination station which allows the patient **302** to be comfortably positioned for the measurement of the eye **120**, as earlier described. For convenience to the technician operating the apparatus **300**, a computer monitor, mouse, and keyboard are located on a separate cart for this embodiment of the present invention, herein described. The apparatus **300** includes a housing **304** having a platform **306** which is carried by a rigid frame **308**. The frame **308** includes wheels **310** to facilitate shipping and installation at the clinical site, as well as locking and leveling feet **312** for securing the apparatus to the supporting floor **314**. Once the apparatus is positioned, the integrated leveling feet **312** are deployed to provide a stable stationary frame **308**, and thus platform **306**.

The operation of this apparatus is described in the aforementioned EP 1153570.

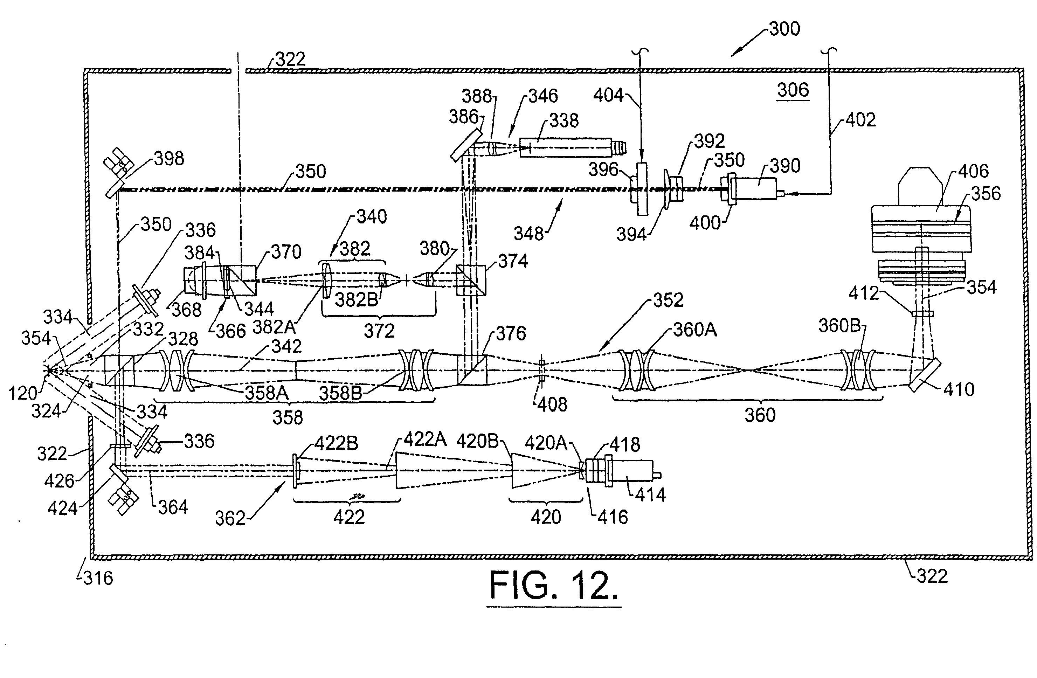

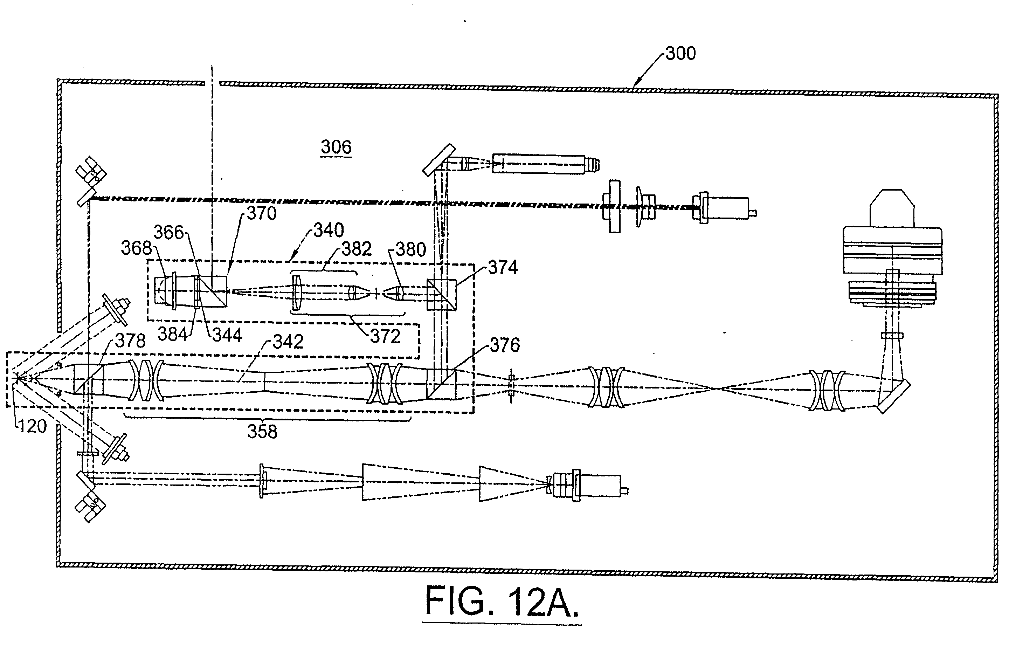

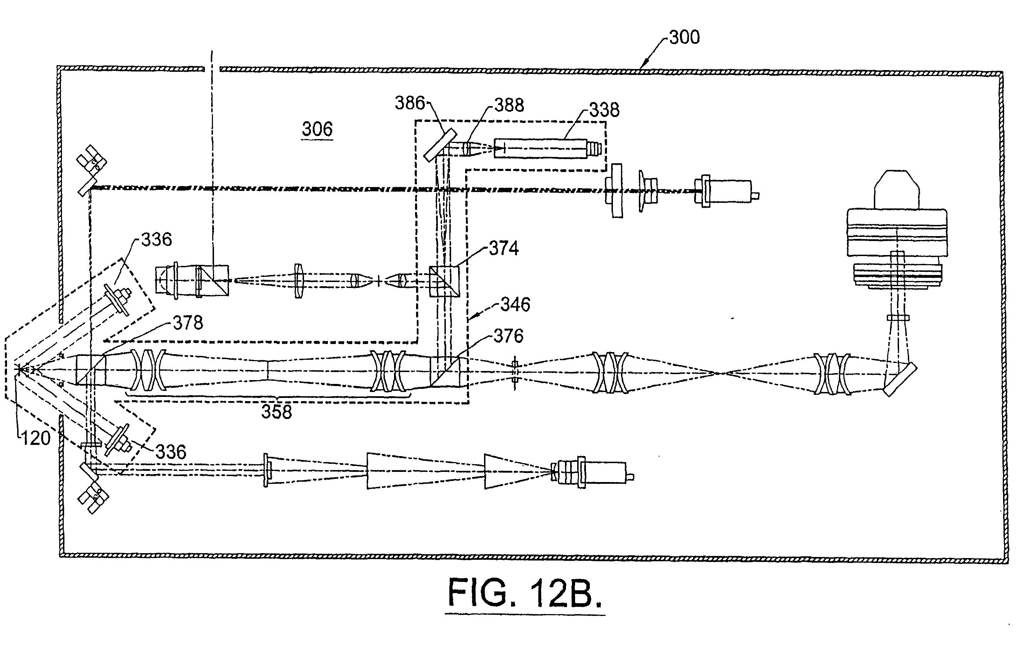

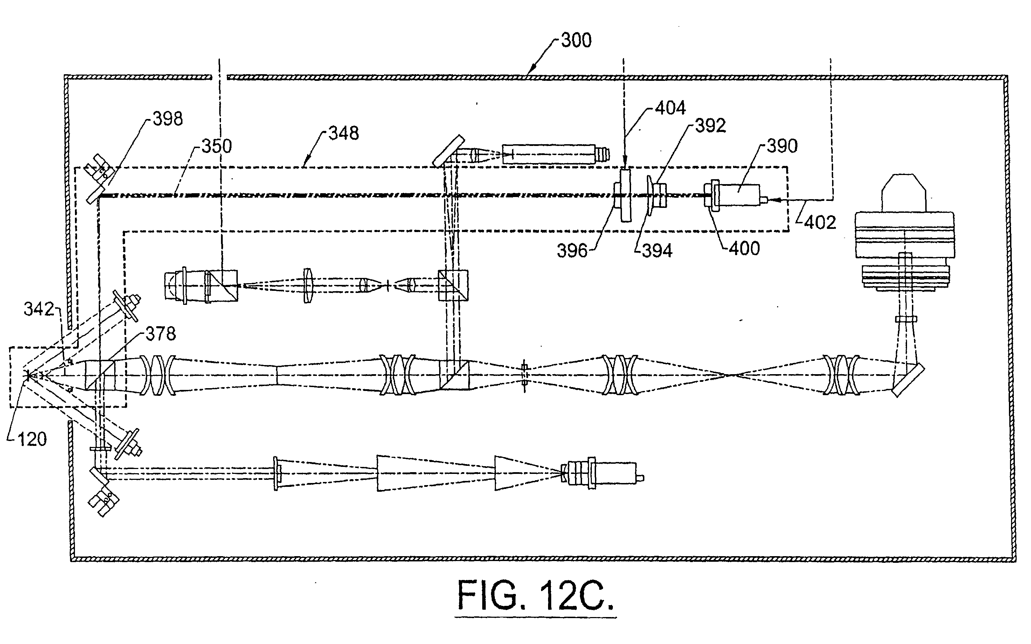

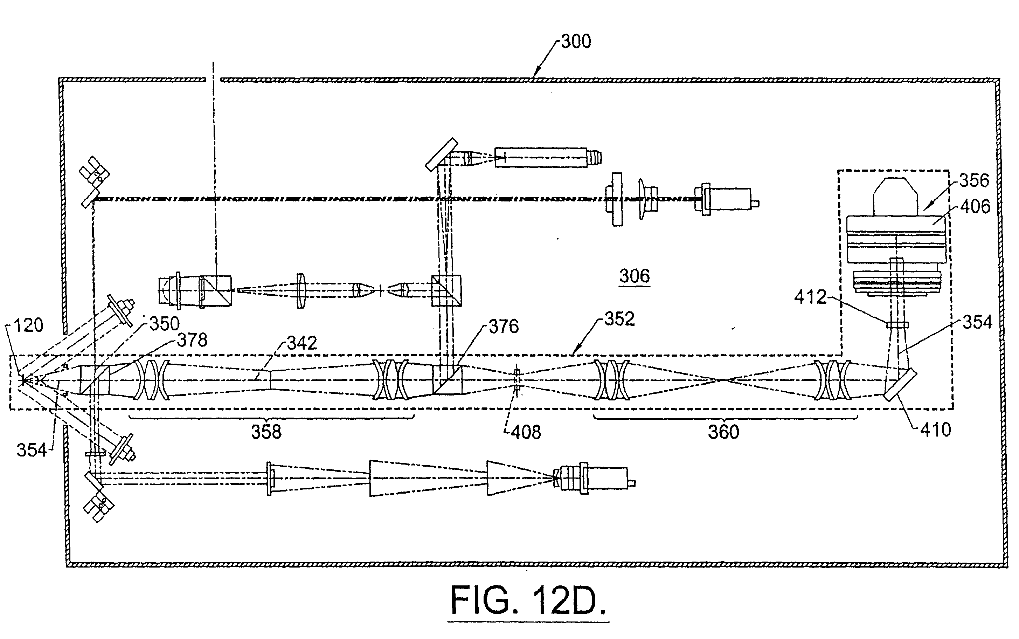

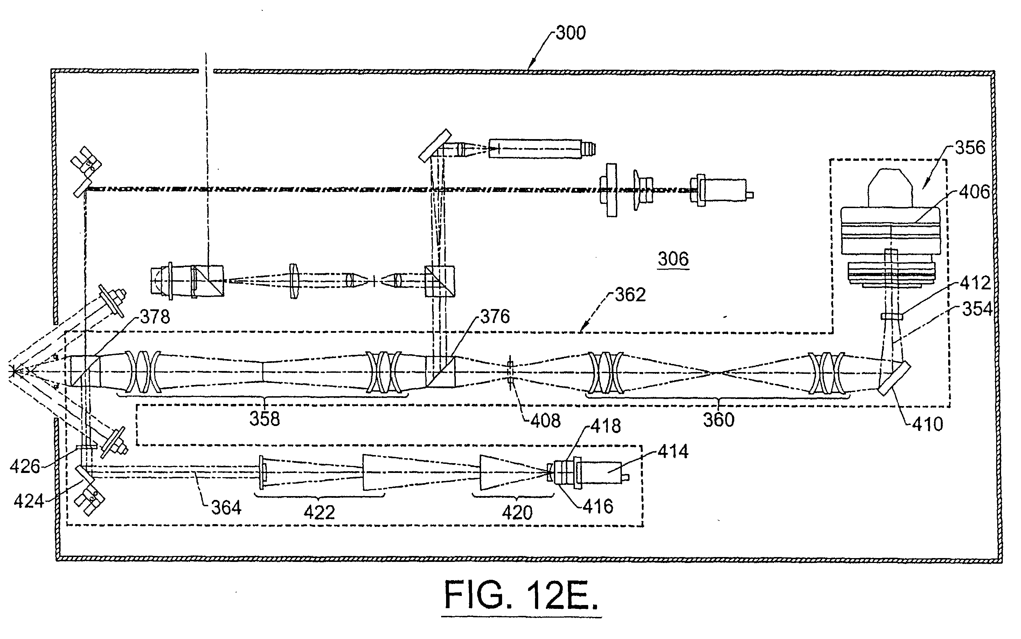

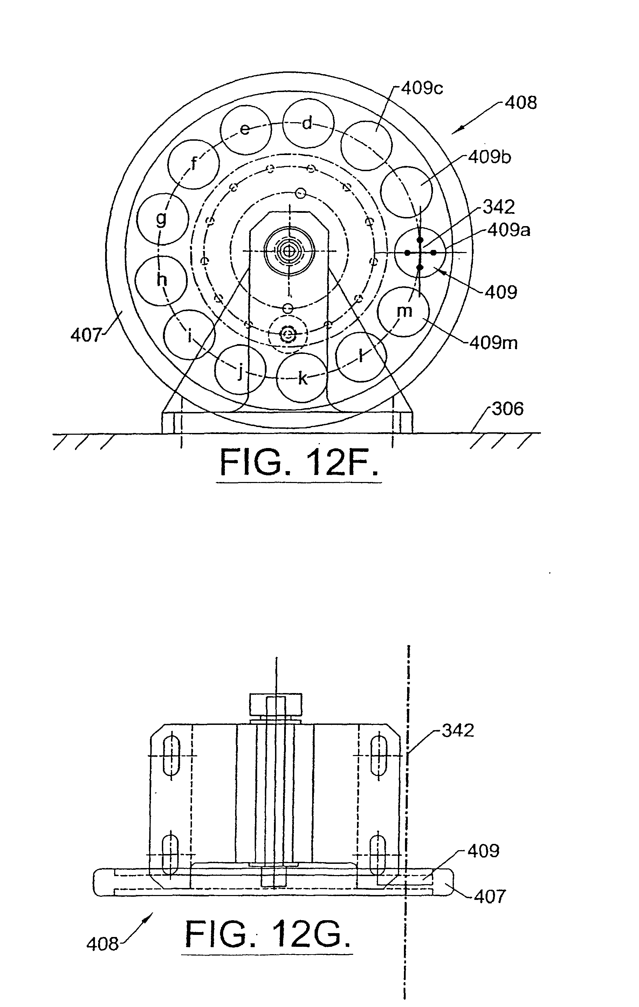

With reference to FIG. 12, a fourth optical path **352**, a re-emitted wavefront optical path illustrated in isolation in FIG. 12D for convenience to the reader, conveys the reflected wavefront **24** of FIG. 2, and herein described with numeral **354** re-emitted from the eye **120** and directed towards a wavefront sensor **356**. To accomplish this, first and second afocal relay stages **358, 360** transfer the reflected wavefront **354** from the corneal plane of the eye **120** to the entrance face of the wavefront sensor **356**. Finally, with reference again to FIG. 12, a fifth optical path **362**, a calibration wavefront optical path illustrated in isolation in FIG. 12E for convenience to the reader, injects collimated laser light into the wavefront transfer path leading to the sensor **356**. Software operable within the computer **326**, described earlier with reference to FIG. 9, uses collimated light wavefront sensor output data to calibrate the apparatus 300 prior to patient measurement.

With continued reference to FIGS. 12, and 12A, the first optical path **340** is herein described as a fixation path which provides a reference image to the patient, such that the eye **120** is properly aligned when the patient is fixating on the reticle **344** of a reference target **366**. A target illumination lamp **368** backlights the fixation target **366**, which fixation target image reaches the patient eye **120** by transmission through a 50/50 beam splitter **370,** lenses **372,** reflection in 50/50 beam splitters **374, 376**, and transmission through lens combinations of afocal relay stage **358**, as well as through polarizing beam splitter **378**. In addition, a spectral filter is placed over the target illumination lamp **368** to remove radiation over the 620-790 nm wavelength range that might otherwise interfere with a wavefront measurement at 670 nm. The lens combinations in the first relay stage **358** contain identical lens elements mounted in reverse order. Each consists of two meniscus lens elements, with an interposed achromatic doublet. The lens combinations work in tandem as a unity magnification afocal relay stage.

The optical elements including the polarizing beam splitter **378**, the lenses of the first afocal stage **358,** the beam splitters **374, 376**, and one lens **380** of the lenses **372** are mechanically fixed in place on the surface of the platform **306**. The optical elements including a lens pair **382** of the lenses **372**, the beam splitter **370**, the fixation target **366**, and the illumination lamp **368** are all mounted on one precision linear translation stage, capable of movement along the optical axis **342** of this pathway. Translation of these optical elements focuses the fixation target **366** for the patient's view, compensating for any myopia/hyperopia present in the eye **120**. During patient examination the focus translation stage is adjusted to place the target optically just beyond the eye's infinity focal plane. This allows the patient to see a relatively distinct reticle pattern without stimulating accommodation by the eye **120**. The beam splitters **378, 376, 374** serve as interfaces between other optical pathways within the optical axis **342**, as will herein be described in further detail. By way of example, the beam splitter **370** is included for alignment purposes. A photo-detector **384** attached to the center of the left edge of beam splitter **370** senses light transmitted toward the fixation target along the optical axis.

With reference again to FIGS. 12 and 12B, the second optical path **346** captures video images of the eye **120** at an examination plane. This allows the clinical operator/technician to assist in patient alignment, and to measure actual eye displacement during the wavefront measurement. As earlier described, the illumination lamps **336** illuminate the eye **120**. The image of the eye is conveyed to the video camera **338** by transmission through the polarizing beam splitter **378** and the lens combinations **358,** reflection in the 50/50 beam splitter **376,** transmission through the 50/50 beam splitter **374,** reflection off mirror **386,** and transmission through lens **388**. All these optical elements are fixed in place on the surface of the platform **306.** By way of example, this second path **346** provides a video field of view approximately 22 mm in diameter at the eye plane, with a limiting resolution of ~64 mm. As earlier described, a number of filters are placed in front of each eye illumination lamp **336** to reduce the spectral bandwidth of the radiation reaching the eye **120**. By way of example, these will includes a blue filter to remove light at wavelengths below ~455nm (for eye safety), an infrared filter to remove light at wavelengths above ~920nm (for eye safety), and a rejection filter to remove light over the wavelength range 620nm - 790nm (to prevent interference with the wavefront measurement at 670 nm).

With continued reference to FIGS. 12 and 12C, the third optical path **348** irradiates a small spot on the patient's retina with eye safe laser radiation, as earlier described with reference to FIGS. 1A - 1D. The irradiated retinal spot on the fovea centralis **123** of the retina **122** is, as herein described, the origin of the re-emitted wavefront **130** measured by the sensor **356**. The output beam, probe laser beam **350** from diode laser **390** reaches the patient eye **120** by transmission through a linear polarizer and attenuator **392**, lens **394**, shutter **396**, and reflection off mirror **398** and in the polarizing beam splitter **378**. All these elements are fixed in position.

In one embodiment of the present invention, output of the diode laser **390** is essentially collimated and is focused onto a corneal surface of the eye **120** by lens **394**. As described in U.S. Patent No 6270221 for "Apparatus And method For measuring Vision Defects Of a Human Eye," the projected probe laser beam **350**, collimated light from the diode laser **390**, is directed by a long focal length lens **394** for focusing on the anterior surface of the cornea **126** of the eye **120**, as illustrated by way of example with reference again to FIG. 1B, passing through the pupil and lens **124** of the eye **120**, and onto the retina **122** as a small measurable spot on the fovea centralis **123.** In one embodiment, the lens **394** comprises a zoom lens for varying the focus and moving the focus location as desired. By focusing on the cornea **126,** the measurement is minimally dependent on the curvature of the cornea. However, other locations proximate the corneal surface are acceptable.

While diffraction and various aberrations are present, the present invention avoids the aberration effects from the cornea which typically dominate. The lens of the eye **120** contributes a relatively small aberration effect when compared to that of the cornea **126**. Further, and with regard to the selection of the lens **394**, selecting a lens with a short focal length would provide a relatively large incident angle of the beam **350,** a well focused point on the surface of the cornea **126**, and less aberration effects from the cornea. A small incident angle provides a larger focus point on the cornea **126**, but a more desirable smaller spot on the retina **122**, which spot size will depend on the wavelength and starting point size and focal length of the lens **394** selected. Embodiments of the present invention including lenses of approximate one half meter and 100 mm, by way of example, haves been effectively used.

In one embodiment, as herein described by way of example, each of the lenses of the second afocal relay stage **360** consists of three lens elements, two meniscus lenses and an interposed achromatic doublet. However, they are not identical, and their combined action serves to magnify the passing wavefront **130**. The wavefront **354** at the trial lens holder **408** location is imaged onto the surface of the microlens array **412** with a magnification of 1.22. Magnification of the wavefront image by this defined factor of 1.22 reduces the wavefront slope at each point in the image plane by the same 1.22 factor. This extends the measurement dynamic range of the device, again without decreasing accuracy. In addition, this magnification distributes the wavefront **130** over more elements, CCD cells **38** as earlier described with reference to FIG. 6, in the microlens array **412**, thus increasing the number of slope measurements provided by the wavefront sensor **356**. The mirror **410** is included to fit elements of the apparatus **300** within the dimensions of the platform **306**. In addition, the mirror **410** also allows optical alignment adjustment for the microlens array **412** and the CCD camera **406** combination. As earlier described, by way of example, with reference to FIGS. 3-6, the microlens array contains a square array of microlenses which divide the incident wavefront into a transverse array of secondary "wavelets." These wavelets are focused onto a detector surface of the CCD camera, which is positioned parallel to the microlens array and one focal length posterior thereto. The pattern of focused wavelets in the CCD image is used to calculate the shape of the incident wavefront.

As illustrated with reference again to FIGS. 12 and 12E, the calibration beam path **362** provides the collimated beam **364** to the Hartman Shack wavefront sensor **356.**

This is further described in EP 1153570.

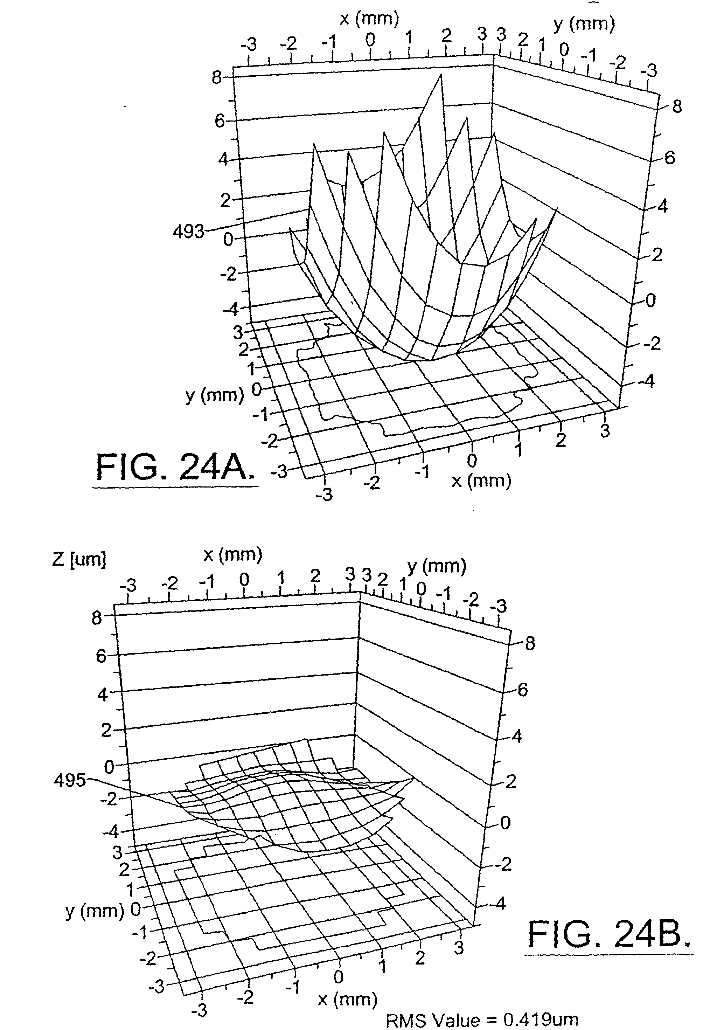

An example of the computed Zernike coefficients for an eye and the corresponding wavefront reconstruction **493** is illustrated with reference to FIG. 24A. By way of example, for the wavefront illustrated with reference to FIG. 24A, the spherical and cylindrical powers computed from the wavefront are -1.60/-1.13 x 150.4. The corresponding values obtained by an optometrist performing a phoropter examination (converted to the corneal plane) were -1.47/-1.19 x 150. The standard measurements of spherical and cylindrical powers agree well with the computation of spherical and cylindrical powers, but there are also higher order aberrations present. By way of further example, FIG. 24B illustrates just these higher order aberrations **495** on the same scale as the plot of FIG. 24A.

FURTHER DISCLOSURE OF CLAIMED FEATURES OF THE PRESENT INVENTION

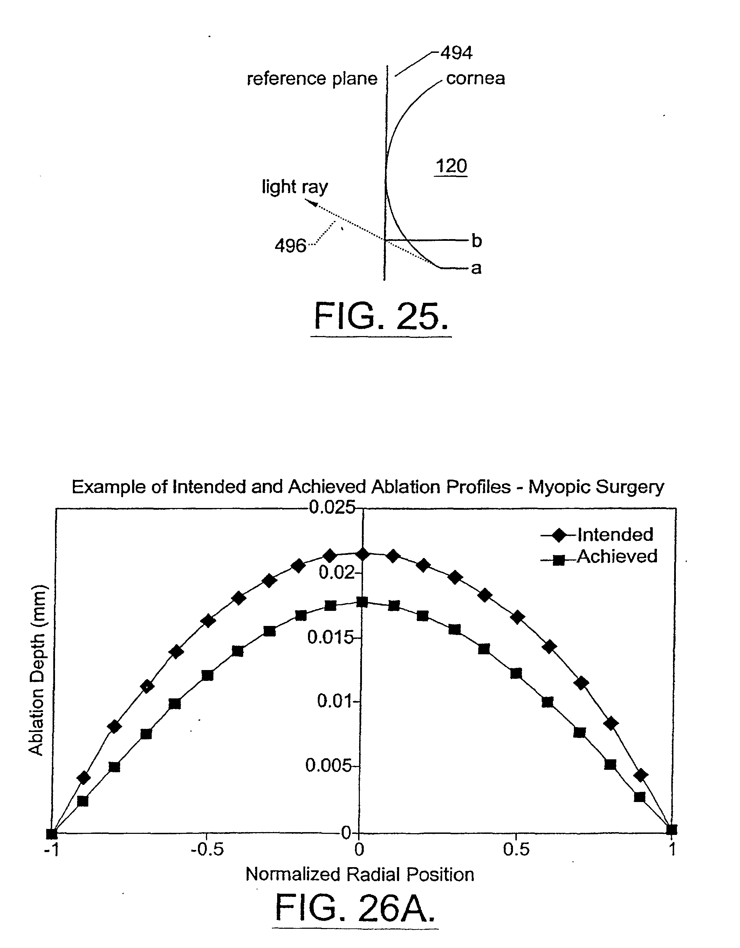

With regard to the optical path difference (OPD), scaling an optical path difference profile, OPD(x,y), by a refractive index difference (cornea to air) is not the only step included to calculate the correct ablation profile. In addition, the present invention allows for a treatment on the curved corneal surface, while the wavefront measurement was made at a plane tangent to the cornea, as illustrated with reference to FIG. 25, which is exaggerated to illustrate the effect. The image plane of the wavefront path is the lenslet array plate. The object plane of the wavefront path is the reference plane **494**. In this highly-exaggerated myopic case, herein described by way of example, one light ray **496** emerging from the eye **120** at transverse location a is detected at a transverse location b. The wavefront reconstructed from sensor data will have the slope of this ray at location *b*. Although this is true of the wavefront at the reference plane **494**, simple scaling of this wavefront would yield an ablative treatment at corneal location *b* that may not be entirely correct. In actuality this effect is small. The radius of curvature of the cornea is typically on the order of 7.5 mm. (a range of 7-8 mm encompasses most eyes.) At a transverse location 3 mm from the corneal apex, the distance from the corneal surface to the reference plane is only ~0.63 μm. For a 10 diopter myope, a light ray exiting the cornea at a = 3.0 mm will cross the reference plane at b = 2.98 mm. The difference between a and b in this example is only 20 μm. Although small this geometric effect is systematic, having progressively greater impact on the measurement with increasing radial distance from the corneal apex. To increase the accuracy of the treatment profile, compensating for the curved geometry may be performed in the following manner:

1. Wavefront slopes are calculated at each measurement point in the reference plane.
2. The cornea is assumed to have a nominal radius of curvature (~7.5 mm).
3. The wavefront slopes measured at the reference plane is projected back onto the nominally curved cornea. The wavefront is measured to have a certain slope at b in the reference plane, described above. It is a straightforward mathematical process to calculate the point a where this ray exited the cornea.
4. The wavefront is reconstructed based on the measured slopes at the calculated corneal locations. This wavefront is used in determining the ablation profile.

As above described, a wavefront measurement has the patient correctly positioned at the apparatus **300**. The eye **120** being measured is at the correct location and looking in the appropriate direction. Based on analysis of the allowable eye-positioning tolerances, the apparatus **300** of this embodiment of the present invention provides the following patient position information:

The capability for ensuring that the subject eye is at the right location along the longitudinal (z) axis of the apparatus with an accuracy of +/- 1 mm.

The capability for ensuring that the subject eye is correctly positioned laterally with respect to the apparatus (i.e., in x-y) with an accuracy of +/- 1 mm.

The capability for ensuring that the subject eye is correctly positioned in angle with respect to the apparatus (i.e., the difference between the visual axis and the optical axis of the system) with an accuracy of +/- 0.5 degrees.

The capability for aligning an on-screen reticule to a set of marks applied to the eye outside the limbus to record the rotational orientation of the eye (i.e., about z) with respect to the apparatus with an accuracy of +/- one degree.

Once in position, the patient's eye can be successfully examined by the wavefront sensing technique. This embodiment of the apparatus includes a sufficient dynamic range to measure eyes over the expected scope of refractive errors. In addition, the apparatus detects complex aberrations, and does so with sufficient accuracy to serve as the basis for ablative treatment.

The following list provides range and accuracy parameters, by way of example, for clinical wavefront measurements that can be obtained by this embodiment of the apparatus. This list is provided by way of illustration and does not limit the scope of the present invention.

1. capable of measuring wavefronts with spherical refractive powers in the range +6 to -15 diopters and cylindrical powers in the range 0 to -6 diopters.
2. capable of measuring coma and spherical aberration.
3. capable of measuring refractive errors over a pupil zone of up to 8 mm in diameter.
4. able to measure the refractive errors within the specified ranges to an accuracy of 0.042 $\mu$m RMS in air.

A computation of a shot pattern is performed in the LADARVision® system. The Zernike coefficients computed in the manner described here are imported into the LADARVision® system along with all other d measurement and patient information and used along with LADARVision® system parameters to compute the optimal number and placement of shots.

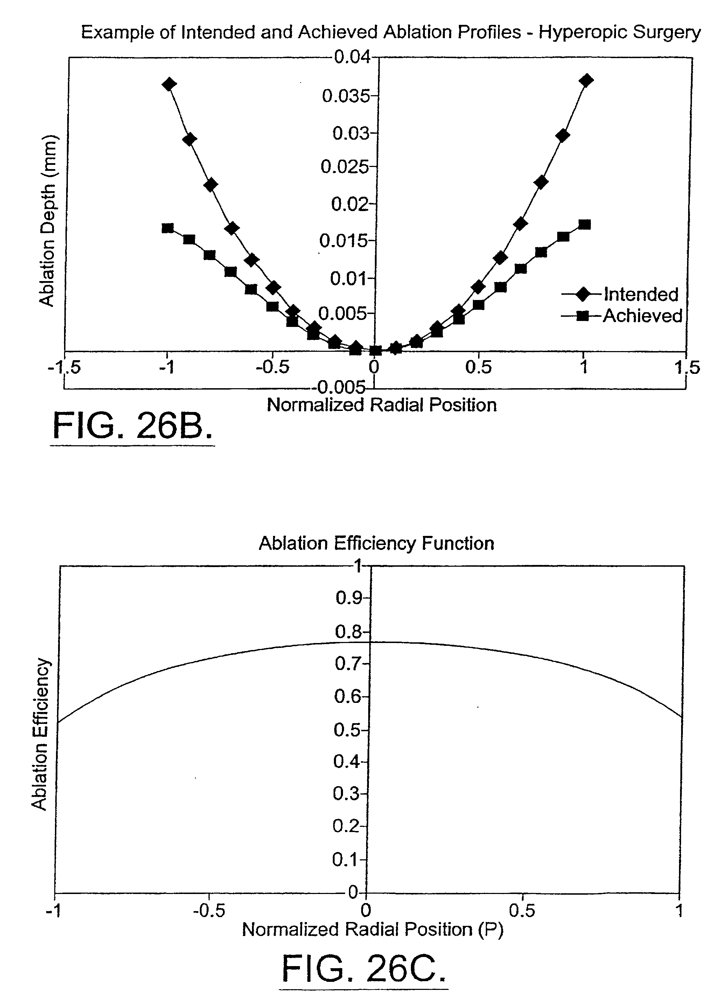

One embodiment of the present invention for a calculation of a treatment laser spot pattern includes an ablation effectiveness distribution over the corneal surface. One embodiment of the present invention, as herein described, optimizes refractive surgery ablation profiles so that post operative aberrations are minimized. One treatment profile takes into account information beyond just that of pre-operative aberrations. As the reader will appreciate, the use of wave front measurement devices has provided greater insight into the effectiveness of current excimer ablation profiles. Analysis of multiple patients for pre and post laser reflective surgery has resulted in a model for describing an effectiveness of a laser ablation as a radially symmetric attenuation function. One embodiment of the present venture provides for this attenuation function. As illustrated by way of example with reference to FIGS. 26A and 26B, a difference exists between an intended change in corneal depth using laser ablation, and an achieved change. FIG. 26A illustrates an intended and achieved profile for surgery on a myopic eye, while the 26B illustrates an intended and achieved profile for surgery on a hyperopic eye. The ablation depth versus normalized radial profile plots of FIGS. 26A and 26B are representative of multiple surgeries analyzed. A constant attenuation independent of radial position results. Sometimes the attenuation is zero. In addition, a radially symmetric attenuation function results. Such a function can be described by an equation of the form: Ablation Efficiency$(\rho)$ = A$\{1+B\rho^2 + C\rho^2\}$,

where $\rho$ is a normalized radial position, and A, B, and C are coefficients describing the attenuation function. The attenuation function may be graphically described, by way of example, with reference to FIG. 26C. As a result, an embodiment of the present invention takes a previously unknown efficiency or attenuation function and modifies treatment profiles accordingly so that a desired outcome is achieved. By way of illustration and example, this may be accomplished by taking a desired change in corneal depth (e.g. a nominal ablation profile), and dividing the nominal profile by the attenuation function. This yields a new profile which, when ablation is performed, will result in the desired profile. One approach is to compute the Zernike description of the ablation profile as earlier described, and divide the resulting Zernike polynomial by the attenuation function to compute a modified Zernike description of the ablation profile that is to be used with the ablation laser system. By way of example, if the $P_{DESIRED}$ is the desired change in corneal depth (i.e. the desired achieved ablation profile) and $P_{INPUT}$ is the profile to be entered into the ablation laser system, then $P_{INPUT}$ may be defined by: $P_{INPUT}(\rho,\theta) = P_{DESIRED} \div A\{1+B\rho^2+C\rho^4\}$

With reference again to FIG. 6, and by way of further example, the output from wavefront analyzer **26**, e.g., the Zernike expansion of equation (19), can be used in a variety of ways. For example, the output may be used to continually or periodically monitor the progress or effects of an ophthalmic procedure, with such stored on disc or transmitted via e-mail, and the like. In addition, the measurement of the eye and the resulting surgery need not take place at the same site. The output could also be used to develop an optical correction for the eye **120**. The optical correction will make the aberrated wavefront **130** appear approximately as the planar wavefront **110**. As described above, the optical correction can be implemented in a variety of ways. In each case, the output of the wavefront analyzer **26** is input to a processor 90 which converts the Zemike expansion of equation (19) into a form suitable for being implemented as one of the possible optical corrections. Alternatively, the processor **90** may also be implemented at the processor **40** of the wavefront analyzer **26**, described earlier with reference to FIG. 6.

By way of further example, the processor **90** can be used with preselected Zernike coefficients from the expansion of equation (19) to generate a standard sphero-cylindrical correction for a lens grinder **92** to produce a conventional optical lens, e.g., a lens for glasses, a contact lens, and the like.

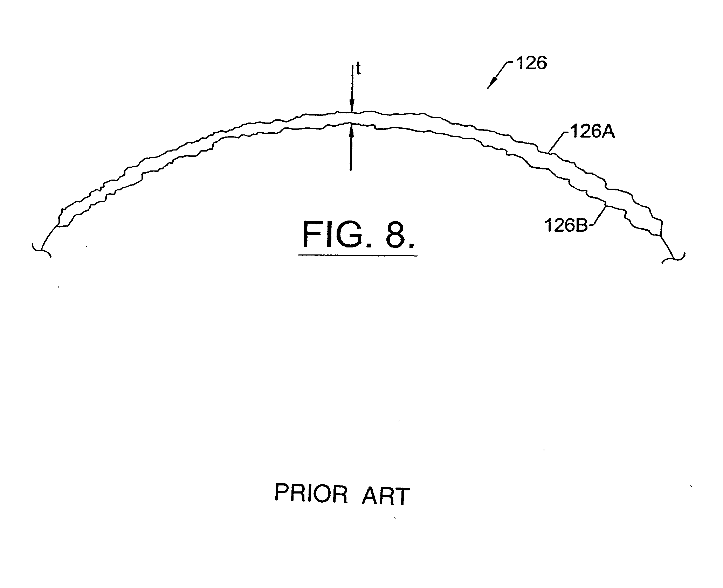

In one embodiment of the present invention, herein presented by way of example, the processor **90** includes a modification of the Zernike reconstruction of the aberrated wavefront **130** by the index of refraction of the cornea **126** minus that of air, having value of **1**, as earlier described, to calculate an amount of corneal material to be ablated at each corresponding (x,y) location on the cornea **126**. This information regarding the amount of corneal material can be used in conjunction with a laser beam delivery system **94** that typically has eye tracking capability. The laser beam delivery system **94** including the eye tracker is placed in line with the optical axis of the apparatus **11**, as illustrated again with reference to FIG. 7. The eye tracker portion allows the apparatus **11** to respond to unwanted eye motion. The system **94** would typically focus short pulses or "shots" of ablating laser light onto the cornea **126** to remove a specified thickness t of material at each location. This is shown diagrammatically in FIG. 8 where the uncorrected surface of the cornea **126** is referenced by numeral **126A** and the corrected surface of cornea **126** after ablation is referenced by numeral **126B**. In accordance with the present invention, the ablation thickness **t** is specified across the aperture of the cornea measured, e.g., the 6 millimeter circle to which the eye's pupil was dilated during the measurement of the eye. Outside the prescribed treatment circle, a tapering blend zone of partial ablation may be added to minimize severe changes in corneal curvature and hence lessen regression. The laser beam delivery system **94** removes thickness t to achieve the optical correction, which results in the corrected cornea surface **126B**. Note that the optical correction is not concerned with the ultimate corneal topography, but instead removes corneal material to achieve an optical correction that takes into account all ocular aberrations of the eye **120**. This is important because the shape of the corneal surface can be independent of the correction d because the eye's vision depends on numerous factors besides corneal curvature. Hence, the best corneal surface topography for optimal vision may be far from regular in that it may compensate for the errors in the eye's other surfaces. Thus, it is apparent that the present invention can be used to provide corneal surface corrections other than the conventional spherical and/or cylindrical corrections.

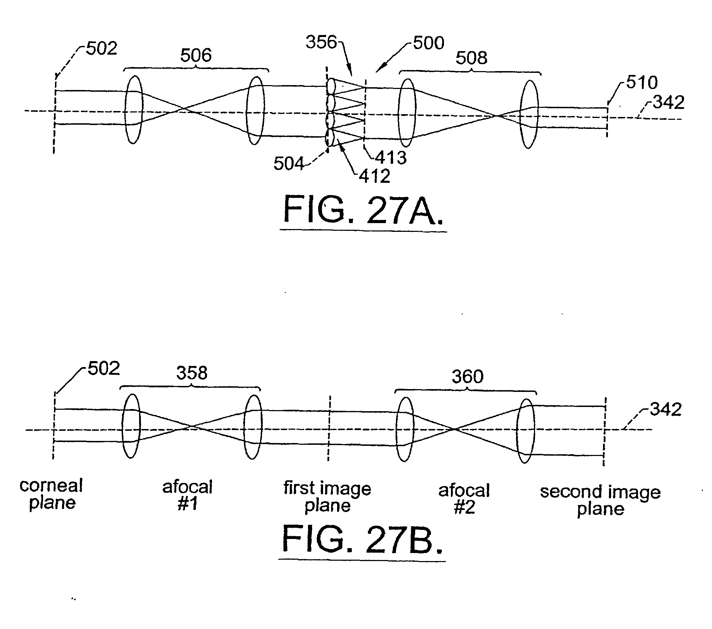

As described earlier with reference to FIG. 12, the apparatus **300** of the present venture includes first and second afocal relays stages **358, 360**. To retain the benefit of wavefront magnification, as a means of increasing the dynamic range of the wavefront sensor **356** to accommodate patients with large refractive errors, while at the same time allowing for incorporation of a small format, inexpensive camera to record the wavefront slope data, a modification **500** to the apparatus **300** as illustrated with reference to FIG. 27A is provided.

By way of example, a lens array may also be positioned and configured as illustrated with reference to FIG. 27B, wherein a portion of the apparatus **300** of FIG. 12 includes the first and second afocal stages **358, 360** within the optical axis **342**, and the wavefront sensor **356** consist of the microlens array and CCD camera separated by a fixed distant, as earlier described with reference to FIG. 6. This optical path through the afocal relay stages results in an image of the corneal plane **502** at the lenslet array, i.e. at the entrance face of the actual wave front sensor **356**. This can be accomplished by a single afocal stage. As earlier described with reference to FIG. 12, the apparatus **300** includes an intermediate image plane as insertion point, the holder **408**, for a trial lens. Placing a spherical lens into the optical axis **342** at the first image plane, in theory, could be used to remove the defocus wavefront error. This would potentially expand the dynamic range of the apparatus **300**. However, the trial lens approach s a moving mechanism that can position lenses at the first image plane with tremendous accuracy in repeatability. It is highly desirable that alternative means be developed to address dynamic range.

One way to accomplish this is to magnify the corneal plane image at the lenslet array with the afocal stage **360**, earlier described. Magnification of the wavefront reduces the wavefront slope, so that the displacement of the focused lights spots on the CCD is decreased. The chosen magnification factor used with the apparatus **300** second afocal stage **360** is approximately 1.2 which is sufficient to cover the desired range in refractive errors. A magnification factor in excess of 1.5 is desirable for expanding the use of the apparatus **300.** However, simply magnifying the corneal plane has a drawback in that it necessitate a large aperture wavefront sensor. That is, both the lens array and the CCD camera preferably have large cross-sectional areas to encompass the magnified image of the point of plane. This is not a significant issue for the lens array. However, a large format CCD camera is quite expensive and such cameras are only available from a limited number of vendors.

To resolve such concerns, the modification **500** illustrated with reference again to FIG. 27A is provided. The corneal plane **502** is imaged at a reference plane **504** by an afocal relay stage **506**, which magnifies the corneal plane by a preselected amount. The lenslet array **412** is placed at the reference plane **504.** Focused spots of light from the eye **120** are produced at the lenslet array focal plane **504**. Rather than place the CCD detector face at the reference plane **504**, an optical train **508** is inserted to image the array focal plane **413** at yet another plane, a final image plane **510**, at which plane the CCD detector face is positioned. The afocal relay stages **358, 360** described earlier with reference to FIGS. 12 and 27B, may or may not be included, as desired. However, the magnification of the array focal plane at the final image plane **510** is provided. This allows a small, relatively inexpensive, active area camera to be used as the light recording element in the wavefront sensor. Details of optical design including magnification specifics can be adjusted to maximize performance for a given camera and lens array plate specification.

The advantages of the present invention are numerous. A totally objective approach is presented for measuring ocular aberrations. The approach is effective for a wide range of vision defects. Accordingly, the present invention will be of great utility in a wide variety of clinical applications. For example, the calculated Zernike coefficients can be used to develop a completely objective lens prescription or a corneal correction that could be accomplished with laser ablation. In addition, each of the wavefront sensor embodiments provides for a greater degree of accuracy over the prior art with respect to measuring wavefront deflections. Further, the present wavefront sensor can be adjusted in terms of gain simply by adjusting the separation distance between the imaging plane of the sensor and the planar array of light-sensitive cells.

The objective measurement of the present invention will also find great utility for a large variety of applications where the "patient" is unable to provide feedback as d by conventional eye diagnosis. For example, the present invention could be used to evaluate the eyes of any patient not possessed of demonstrative communicative skills, e.g., babies, animals, dead specimens, as well as any constructed optical system, since the present invention is an objective analysis not requiring any assessment from the "subject". All that is necessary is for the subject's eye to be properly positioned so that proper optical access to the eye can be obtained.

The present invention will also be used in the area of identification should it be determined that each eye's Zemike coefficients are unique. Then, the present invention would find great utility in the fields of law enforcement, credit card/bank security, or any other field where positive identification would be beneficial.

Although the invention has been described relative to a specific embodiment thereof, there are numerous variations and modifications that will be readily apparent to those skilled in the art in light of the above teachings. It is therefore to be understood that, within the scope of the appended claims, the invention may be practiced other than as specifically described.

**Claims**

1. An optical correction system (300) for determining a correction of visual defects of an eye, the optical correction system comprising:

> an energy source for generating a beam of optical radiation;
> focusing optics disposed in the path of the beam for directing the beam through the eye (120), wherein the beam is reflected back from the retina of the eye as a wavefront (130) of radiation emanating from the eye;
> a wavefront analyzer (26) disposed in the path of the wavefront emanating from a cornea (126) of the eye for determining an optical path difference between a plane wave (110) and the wavefront; and
> a converter (40, 90) for providing an optical correction which, if placed in the path of the wavefront, causes the wavefront to appear approximately as a plane wave,
> means to compensate for the curved geometry of the cornea comprising:

> - means to measure wavefront (130) slopes at a reference plane (131),
> - means to project said measured wavefront slopes back onto points of origin on the cornea of the eye assumed to have a nominal radius of curvature,

- means to calculate a point where said beam exits the cornea, and
- means to reconstruct the wavefront based on the measured slopes at the calculated corneal locations,

**characterized in that** said compensation means comprises;
means for calculating a radially symmetric attenuation function represented by

$$A\{1 + B\rho^2 + C\rho^4\},$$

where $\rho$ is a normalized radial position, and A, B and C are coefficients describing the attenuation function, and the system further includes first and second a focal relay stages (358, 360) disposed in the path of the wavefront emanating from the cornea, the first and second a focal relay stages in combination with a microlens array being adapted to effect a magnification of the wavefront, thereby increasing the dynamic range of the system.

2. A system according to Claim 1, wherein the optical correction is a provided by a lens prescription (92).

3. A system according to Claim 1, wherein the optical correction is a provided by means (94) to ablate an amount of corneal material from the eye.

4. A system according to Claim 3, wherein the ablation means comprises a laser beam (14, 18) delivery system (94) for bombarding the eye with a laser beam having power sufficient for ablating corneal material of the cornea (126) of the eye, and wherein the optical correction is achieved by the removal of an amount of corneal material.

5. A system as in Claim 4, wherein the laser beam delivery system (94) includes an eye tracker (25) for monitoring motion of the eye and for adjusting the positions of the laser beam responsive to the motion.

6. A system as in Claim 3, wherein the optical correction is a prescribed alteration of corneal surface curvature of the eye (120), and wherein the optical correction achieved by the reshaping of the corneal surface curvature of the eye is based on the prescribed alteration without regard to a resulting topography of the overall surface of the cornea.

7. A system according to Claim 1, wherein the converter (40, 90) provides the path difference from a Zernike reconstruction of the wavefront (24, 30) and wherein the path difference is divided by a difference between an index of refraction of corneal material and an index of refraction of air.

**Patentansprüche**

1. Optisches Korrektursystem (300) zur Bestimmung einer Korrektur visueller Defekte eines Auges, wobei das optische Korrektursystem umfaßt:

- eine Energiequelle zur Erzeugung eines Strahls optischer Strahlung;
- eine Fokussierungsoptik, die im Pfad des Strahls angeordnet ist, um den Strahl durch das Auge zu richten (120), wobei der Strahl von der Netzhaut des Auges als eine Wellenfront (130) von vom Auge ausgehender Strahlung zurück reflektiert wird;
- eine Wellenfrontanalysevorrichtung (26), die im Pfad der von einer Hornhaut (126) des Auges ausgehenden Wellenfront zur Bestimmung einer optischen Wegdifferenz zwischen einer ebenen Welle (110) und der Wellenfront angeordnet ist; und
- einen Wandler (40, 90) zum Erzeugen einer optischen Korrektur, der, wenn er im Pfad der Wellenfront angeordnet ist, bewirkt, daß die Wellenfront näherungsweise als eine ebene Welle erscheint,
- Mittel zur Kompensation der gekrümmten Geometrie der Hornhaut, umfassend:

  - Mittel zur Messung von Steigungen der Wellenfront (130) an einer Referenzebene (131),
  - Mittel zur Projektion der gemessenen Steigungen der Wellenfront zurück auf Ursprungspunkte auf der Hornhaut des Auges, von welchen angenommen wird, daß sie einen nominalen Krümmungsradius aufweisen,
  - Mittel zur Berechnung eines Punktes, an dem der Strahl aus der Hornhaut austritt, und
  - Mittel zur Rekonstruktion der Wellenfront basierend auf den gemessenen Steigungen an den berechneten

# EP 1 210 003 B1

Orten der Hornhaut;

**dadurch gekennzeichnet, daß** das Kompensationsmittel umfaßt:

- Mittel zur Berechnung einer radialsymmetrischen Abschwächungsfunktion, die dargestellt ist durch

$$A\{1 + B\rho^2 + C\rho^4\},$$

wobei $\rho$ eine normalisierte radiale Position ist, und A, B und C Koeffizienten sind, welche die Abschwächungsfunktion beschreiben, und wobei das System des weiteren erste und zweite afokale Verbindungsstufen (358, 360) beinhaltet, die im Pfad der von der Hornhaut ausgehenden Wellenfront angeordnet sind, wobei die erste und die zweite afokale Verbindungsstufe in Kombination mit einem Mikrolinsenfeld dazu angepaßt sind, eine Vergrößerung der Wellenfront zu bewirken, wodurch der dynamische Bereich des Systems erhöht wird.

2. System nach Anspruch 1, wobei die optische Korrektur durch eine Linsenvorschrift (92) erreicht wird.

3. System nach Anspruch 1, wobei die optische Korrektur durch Mittel (94) zur Abtragung von Homhautmaterial vom Auge erreicht wird.

4. System nach Anspruch 3, wobei das Abtragungsmittel ein System (94) zur Versorgung mit einem Laserstrahl (14, 18) umfaßt, um das Auge mit einem Laserstrahl zu bombardieren, der eine ausreichende Leistung aufweist, um das Hornhautmaterial von der Hornhaut (126) des Auges abzutragen und wobei die optische Korrektur durch die Entfernung von Hornhautmaterial erreicht wird.

5. System nach Anspruch 4, wobei das System (94) zur Versorgung mit einem Laserstrahl eine Augenverfolgungseinrichtung (25) zur Beobachtung der Bewegung des Auges und zum Anpassen der Positionen des Laserstrahls in Reaktion auf die Bewegung umfaßt.

6. System nach Anspruch 3, wobei die optische Korrektur eine vorgeschriebene Änderung der Oberflächenkrümmung der Hornhaut des Auges (120) ist, und wobei die optische Korrektur, die durch die Neuformung der Oberflächenkrümmung der Hornhaut des Auges erreicht wird, auf der vorgeschriebenen Änderung ohne Rücksicht auf eine resultierende Topographie der Gesamtoberfläche der Hornhaut basiert.

7. System nach Anspruch 1, wobei der Wandler (40, 90) die Wegdifferenz mit einer Zernike-Rekonstruktion der Wellenfront (24, 30) bereitstellt und die Wegdifferenz durch die Differenz zwischen einem Brechungsindex des Homhautmaterials und einem Brechungsindex von Luft geteilt wird.

## Revendications

1. Système de correction optique (300) pour déterminer une correction de défauts visuels d'un oeil, le système de correction optique comportant :

une source d'énergie pour générer un faisceau de rayonnement optique,
des optiques de focalisation positionnés dans le trajet du faisceau pour diriger le faisceau à travers l'oeil (120), le faisceau étant réfléchi par la rétine de l'oeil sous forme d'un front d'onde (130) de rayonnement émanant de l'oeil,
un analyseur de front d'onde (26) positionné dans le trajet du front d'onde émanant d'une cornée (126) de l'oeil pour déterminer une différence de trajet optique entre une onde plane (110) et le front d'onde, et
un convertisseur (40, 90) pour permettre une correction optique qui, si placé dans le trajet du front d'onde, amène le front d'onde à apparaître approximativement sous la forme d'une onde plane,
des moyens pour compenser la géométrie incurvée de la cornée comportant :

- des moyens pour mesurer des pentes de front d'onde (130) dans un plan de référence (131),
- des moyens pour reprojeter lesdites pentes de front d'onde mesurées en retour sur des points d'origine sur la cornée de l'oeil supposée avoir un rayon de courbure nominal,
- des moyens pour calculer un point où ledit faisceau sort de la cornée, et

20

- des moyens pour reconstruire le front d'onde sur la base des pentes mesurées aux emplacements cornéens calculés,

   **caractérisé en ce que** lesdits moyens de compensation comportent :

   des moyens pour calculer une fonction d'atténuation radialement symétrique représentée par

$$A\{1 + B\rho^2 + CP^4\},$$

   où $\rho$ est une position radiale normalisée, et A, B et C sont des coefficients décrivant la fonction d'atténuation, et le système comporte de plus des premier et second étages de relais afocaux (358, 360) positionnés dans le trajet du front d'onde émanant de la cornée, les premier et second étages de relais afocaux en combinaison avec un réseau de microlentilles étant adaptés pour effectuer un grossissement du front d'onde, en augmentant ainsi la plage dynamique du système.

2. Système selon la revendication 1, dans lequel la correction optique est fournie par une prescription de lentille (92).

3. Système selon la revendication 1, dans lequel la correction optique est fournie par des moyens (94) destinés à faire l'ablation d'une quantité de matériel cornéen depuis l'oeil.

4. Système selon la revendication 3, dans lequel les moyens d'ablation comportent un système de libération (94) de faisceau laser (14, 18) pour bombarder l'oeil à l'aide d'un faisceau laser ayant une puissance suffisante pour effectuer l'ablation d'un matériel cornéen de la cornée (126) de l'oeil, et dans lequel la correction optique est obtenue par l'élimination d'une quantité de matériel cornéen.

5. Système selon la revendication 4, dans lequel le système de libération de faisceau laser (94) comporte un système de suivi d'oeil (25) pour surveiller un mouvement de l'oeil et pour ajuster les positions du faisceau laser en réponse au mouvement.

6. Système selon la revendication 3, dans lequel la correction optique est une altération prescrite de la courbure de surface cornéenne de l'oeil (120), et dans lequel la correction optique obtenue par la remise en forme de la courbure de surface cornéenne de l'oeil est basée sur l'altération prescrite sans tenir compte d'une topographie résultante de la surface globale de la cornée.

7. Système selon la revendication 1, dans lequel le convertisseur (40, 90) établit la différence de trajet à partir d'une reconstruction de Zernike du front d'onde (24, 30) et dans lequel la différence de trajet est divisée par une différence entre un indice de réfraction du matériel cornéen et un indice de réfraction de l'air.

FIG. 1A.

FIG. 1B.

PRIOR ART

FIG. 1C.

$$\frac{\partial W(x_0, y_0)}{\partial x}$$

FIG. 1D.

PRIOR ART

23

FIG. 2.

PRIOR ART

# FIG. 3.

PRIOR ART

FIG. 4.

PRIOR ART

**FIG. 5.**

PRIOR ART

## FIG. 6.

PRIOR ART

FIG. 7.

PRIOR ART

EP 1 210 003 B1

FIG. 8.

PRIOR ART

FIG. 9.

EP 1 210 003 B1

FIG. 10.

FIG. 11.

FIG. 12.

FIG. 12A.

EP 1 210 003 B1

FIG. 12B.

FIG. 12C.

FIG. 12D.

FIG. 12E.

FIG. 12F.

FIG. 12G.

FIG. 13.

FIG. 14.

EP 1 210 003 B1

1. Perform Reference Measurement ⟋462

2. Select Patient and Eye to Measure ⟋464

3. Align Eye Using Video and Reticles ⟋466

4. Perform Measurement ⟋470

5. Review Eye-System Geometry and Accept or Reject ⟋472

6. Process CCD Image and Accept/Save or Reject ⟋478

7. Process Measurement and compute local slopes ⟋480

8. Generate Reconstructed Wavefront (Optical Path Difference) ⟋492

# FIG. 15.

43

FIG. 16.

FIG. 17.

FIG. 18.

Image invalid because
this section is obscurred

FIG. 19.

FIG. 20.

FIG. 21.

FIG. 22.

Spatial Filter Cross-Section

FIG. 23A.

FIG. 23B.

FIG. 24A.

FIG. 24B.

RMS Value = 0.419um

reference plane

494

cornea

120

light ray

496

b

a

## FIG. 25.

Example of Intended and Achieved Ablation Profiles - Myopic Surgery

Ablation Depth (mm)

Normalized Radial Position

◆ Intended
■ Achieved

## FIG. 26A.

Example of Intended and Achieved Ablation Profiles - Hyperopic Surgery

## FIG. 26B.

Ablation Efficiency Function

## FIG. 26C.

FIG. 27A.

corneal plane     afocal #1     first image plane     afocal #2     second image plane

FIG. 27B.